# EUROPEAN PATENT APPLICATION

(11) **EP 4 768 518 A1**
(43) Date of publication of application: **01.07.2026**
(21) Application number: 24383477.7
(22) Date of filing: 30.12.2024
(51) Int. Cl.: C08G 63/685, A61K 47/59, A61K 47/69, C08G 63/91

(54) **POLYMERS COMPRISING TWO BUILDING BLOCKS OBTAINED BY CLICK CHEMISTRY AND USES THEREOF**

(71) Applicant: Institut Químic de Sarrià CETS Fundació Privada, 08017 Barcelona (ES)
(72) Inventor: FORNAGUERA PUIGVERT, Cristina, 08017 BARCELONA (ES); FAIJES SIMONA, Magda, 08017 BARCELONA (ES); GONZÁLEZ RÍOS, Nil, 08017 BARCELONA (ES); BORRÓS GÓMEZ, Salvador, 08017 BARCELONA (ES); PLANAS SAUTER, Antoni, 08017 BARCELONA (ES)
(74) Representative: ZBM Patents - Zea, Barlocci & Markvardsen

(57) **Abstract**

It relates to a polymer or a salt thereof which comprises a first polymer building block and a second polymer building block wherein: a) the first and second polymer building blocks are covalently bonded through at least a common lateral chain, and b) the first polymer building block is an oligopeptide-modified poly(beta-aminoester). The polymer comprising the two building blocks can be conveniently prepared by click chemistry. It also relates to nanoparticles comprising this polymer, to pharmaceutical and cosmetic compositions comprising the polymer or the nanoparticles, and to their use as a delivery system and in therapy, diagnosis or cell-targeting.

## Description

### Technical Field

The invention relates to polymers comprising two building blocks, in particular two poly(beta-aminoester) (pBAE) building blocks, that are suitable for use in the delivery of active agents. The invention also discloses nanoparticles comprising these polymers, compositions comprising said nanoparticles and methods for their production.

### Background Art

The lack of safe and efficient vectors to deliver active ingredients such as polynucleotides (DNA, RNA and the like) remains the principal handicap for the success of gene therapy. The majority of protocols for polynucleotide delivery employ viral vectors, which are highly efficient delivery systems. However, viral vectors have certain disadvantages, including safety risk, limited capacity to carry polynucleotides and high cost of large-scale production. Non-viral vectors offer potential advantages, including high packing capacity, ease of production, low toxicity and immunogenicity, but are less efficient than viral vectors.

Biodegradable poly(beta-aminoester)s (pBAE)s have been described as potential non-viral polynucleotide delivery vectors capable of condensing both DNA and RNA into discrete nanometric particles.

A continuing need exists for improved non-toxic, biodegradable, biocompatible polymers that can be used to transfect polynucleotides efficiently and that can be prepared economically. Such polymers would be useful in the packaging and delivery of DNA and RNA in gene therapy and for the packaging and delivery of other diagnostic, therapeutic and prophylactic agents or for cell-targeting.

In addition to the above, it is well-known that delivery systems such as nanoparticles (NPs), when are exposed to biological fluids, rapidly adsorb proteins. These form the so-called "hard" and "soft" corona according to their binding strength and exchange rates at the NP surface. The composition of this protein corona depends on many parameters, including particle size, surface chemistry (hydrophilicity, surface charge, etc.), environmental conditions (time, temperature, etc.) and the nature and composition of biological fluids. However, in many *in vivo* applications, the protein corona dictates the fate of nanocarriers and induces fast recognition by the immune system. It, therefore, reduces the circulation lifetime in the blood stream, as well as the targeting efficiency, and influences cellular uptake, biodistribution and toxicity.

In view of the above, there is the need of polymers with improved properties, and which overcome the above identified problems.

### Summary of Invention

The present invention provides a new generation of polymers with improved properties, making them useful in a variety of medical applications, including drug delivery, as well as cosmetic, detergency and surface chemistry applications.

This new technology makes it possible to establish a strategy based on two different building blocks, e.g. two pBAE building blocks, in contrast to a strategy based on a single pBAE polymer such as for example the one disclosed in the PCT application WO2014136100.

In particular, the polymers of the invention comprise a first oligopeptide modified pBAE (OM-pBAE) building block and a second building block which are covalently bonded through at least a common lateral chain. The attachment of the two building blocks through a lateral chain, rather than through their backbone ends, provides more stability to the resulting polymer.

When using this strategy, one of the polymeric building blocks, in particular a pBAE building block, can be functionalized with at least one oligopeptide, and the other building block, in particular also pBAE building block, can be functionalized with a ligand.

Thanks to the use of an oligopeptide-modified pBAE, nanoparticles can be formed under suitable conditions that allow encapsulating compounds of interest, for example genetic material. On the other hand, the functionalization of the second building block with a ligand or cell-targeting moiety makes it possible the interaction with a desired cellular receptor such as for example sugars or mimetics, proteins and peptides or mimetics such as antibodies, or aptamers. Also signaling molecules such as fluorophores may be added. Thus, new delivery systems can be generated with a wide range of different ligands.

Further, the covalent binding of the two building blocks does not negatively affect to the encapsulation efficiency, neither to the suitable size of the nanoparticles to be used as a delivery vehicle. In particular, it was found that the nanoparticles of the invention had low average size, remarkably lower than 1000 nm, as shown in the examples. The above is of relevance because a size below 1000 nm guarantees that the nanoparticles can provide the biologically effect: (a) it is minimized the formation of aggregates, and (b) it is facilitated cell internalization. In addition, small sizes guarantee an appropriate stability when formulated in the form of a pharmaceutical composition and can be easily reproduced.

Furthermore, the polymers of the invention can be readily prepared by a click chemistry reaction between two polymers which will become the two building blocks mentioned above in the final polymer.

As an example, a pBAE polymer functionalized with at least one oligopeptide and a first click functional group may be contacted with another pBAE polymer functionalized with a cell-targeting moiety and a second click functional group, wherein the first and the second click functional groups are click pairs capable to form a cyclic adduct and thus connecting the two initial polymers. For example, the first click functional group may be a strained alkyne or a diene, and the second click functional group may be an azide or a tetrazine, or *vice versa.*

An advantage of such click reactions is that they proceed almost spontaneously without the need for extreme conditions of pH, temperature, or catalysts, so that the reaction can occur when the polymers are dissolved in a medium even when they already form part of the structure of a nanoparticle or other surface or even in physiological media.

In addition, the application of click chemistry allows an easier and more versatile synthesis where the number of ligands per building block, and thus per final polymer obtained after click-chemistry, can be increased since cell-targeting moieties may be present not only in lateral chains but also at the ends of the second building block.

Additionally, as shown in the examples below, the nanoparticles containing the polymers of the invention do not present significant differences with respect to the nanoparticle size or Z potential of the OM-pBAE polymer of the prior art. Furthermore, when myeloid cells o antigen presenting cells, activators of the immune system, were transfected and the cytotoxicity and capacity of the formulations to activate the immune system by presenting mRNA coding for ovalbumin were compared, the results obtained indicated that the new formulations made with the new polymer offer good cell viability and transfection efficiency Thus, in a first aspect the present invention relates to a polymer, or a pharmaceutically acceptable salt thereof, which comprises a first polymer building block and a second polymer building block wherein:
a) the first and second polymer building blocks are covalently bonded through at least a common lateral chain, and
b) the first polymer building block is an oligopeptide-modified poly(beta-aminoester) (OM-pBAE).

A second aspect of the invention relates to a nanoparticle comprising the polymer as defined herein.

A third aspect of the invention relates to a pharmaceutical composition comprising a polymer or a nanoparticle as defined herein, and a therapeutic effective amount of an active ingredient, a cell-targeting ligand, a diagnostic agent, an imaging agent or a combination thereof, together with one or more pharmaceutically acceptable excipients.

A fourth aspect of the invention relates to a cosmetic composition comprising a polymer or a nanoparticle as defined herein, and a cosmetically effective amount of an active ingredient, together with one or more cosmetically acceptable excipients.

A fifth aspect of the invention relates to a polymer or a nanoparticle as defined herein for use as a delivery system.

A sixth aspect of the invention relates to a nanoparticle or a pharmaceutical composition as defined herein, for use in therapy, diagnosis or cell-targeting.

### Brief Description of Drawings

FIG. 1 shows the effect of the addition of Man2-N3-Cap (37) to D1 nanoparticles comprising C6-DBCO-CK3 (33) compared to a KH sample on nanoparticle size (A), and PDI (B).
FIG. 2 shows of the effect of the addition of Man2-N3-Cap (37) to KH and D1 nanoparticles comprising C6-DBCO-CK3 (33) compared to a KH sample on the static light scattering (SLS) over time.
FIG. 3 shows transfection efficiency (A) and uptake (B) upon comparing the flow cytometry data from the different nanoparticles C1, D1 and KH.
FIG. 4 shows cell viability of mo-DC upon being transfected for 24h with pBAE formulations C1 compared with their corresponding controls D1 and KH, respectively.
FIG. 5 shows CD86 upregulation of mo-DC upon being transfected for 24h with pBAE formulations C1, D1, and KH from different generations.
FIG. 6 shows MHC-II upregulation of mo-DC upon being transfected for 24h with pBAE formulations C1, D1, and KH from different generations.

### Detailed description of the invention

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

It is noted that, as used in this specification and the appended claims, the singular forms "a", "an", and "the" include plural referents unless the context clearly dictates otherwise.

For the purposes of the present invention, any ranges given include both the lower and the upper end-points of the range.

### Chemical groups

The term "halogen" (or "halo") includes fluorine, chlorine, bromine and iodine.

The term "alkyl" includes monovalent, straight or branched, saturated, acyclic hydrocarbyl groups. Alkyl is suitably C₁₋₁₀alkyl, or C₁₋₆alkyl, or C₁₋₄alkyl, such as methyl, ethyl, n-propyl, i-propyl or t-butyl groups. Alkyl may be substituted.

The term "cycloalkyl" includes monovalent, saturated, cyclic hydrocarbyl groups. Cycloalkyl is suitably C₃₋₁₀cycloalkyl, or C₃₋₆cycloalkyl such as cyclopentyl and cyclohexyl. Cycloalkyl may be substituted.

The term "alkoxy" means alkyl-O-.

The term "alkylamino" means alkyl-NH-.

The term "alkenyl" includes monovalent, straight or branched, unsaturated, acyclic hydrocarbyl groups having at least one carbon-carbon double bond and, suitably, no carbon-carbon triple bonds. Alkenyl is suitably C₂₋₁₀alkenyl, or C₂₋₆alkenyl, or C₂₋₄alkenyl. Alkenyl may be substituted.

The term "cycloalkenyl" includes monovalent, partially unsaturated, cyclic hydrocarbyl groups having at least one carbon-carbon double bond and, suitably, no carbon-carbon triple bonds. Cycloalkenyl is suitably C₃₋₁₀cycloalkenyl, or C₅₋₁₀cycloalkenyl, e.g. cyclohexenyl or benzocyclohexyl. Cycloalkenyl may be substituted.

The term "alkynyl" includes monovalent, straight or branched, unsaturated, acyclic hydrocarbyl groups having at least one carbon-carbon triple bond and, suitably, no carbon-carbon double bonds. Alkynyl is suitably C₂₋₁₀alkynyl, or C₂₋₆alkynyl, or C₂₋₄alkynyl. Alkynyl may be substituted.

The term "alkylene" includes divalent, straight or branched, saturated, acyclic hydrocarbyl groups. Alkylene is suitably C₁₋₁₀alkylene, or C₁₋₆alkylene, or C₁₋₄alkylene, such as methylene, ethylene, n-propylene, i-propylene or t-butylene groups. Alkylene may be substituted.

The term "alkenylene" includes divalent, straight or branched, unsaturated, acyclic hydrocarbyl groups having at least one carbon-carbon double bond and, suitably, no carbon-carbon triple bonds. Alkenylene is suitably C₂₋₁₀alkenylene, or C₂₋₆alkenylene, or C₂₋₄alkenylene. Alkenylene may be substituted.

The term "heteroalkyl" includes monovalent alkyl groups, for example, C₁₋₆₅alkyl groups, C₁₋₁₇alkyl groups or C₁₋₁₀alkyl groups, in which up to twenty carbon atoms, or up to ten carbon atoms, or up to two carbon atoms, or one carbon atom, are each replaced independently by O, S(O), or N, provided at least one of the alkyl carbon atoms remains. The heteroalkyl group may be C-linked or hetero-linked, i.e. it may be linked to the remainder of the molecule through a carbon atom or through O, S(O)t or N, wherein t is defined below. Heteroalkyl may be substituted.

The term "heterocycloalkyl" includes monovalent cycloalkyl groups in which up to ten carbon atoms, or up to two carbon atoms, or one carbon atom, are each replaced independently by O, S(O), or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, i.e. it may be linked to the remainder of the molecule through a carbon atom or through a nitrogen atom. Heterocycloalkyl may be substituted.

The term "heteroalkenyl" includes monovalent alkenyl groups, for example, C₁₋₆₅alkenyl groups, C₁₋₁₇alkenyl groups or C₁₋₁₀alkenyl groups, in which up to twenty carbon atoms, or up to ten carbon atoms, or up to two carbon atoms, or one carbon atom, are each replaced independently by O, S(O), or N, provided at least one of the alkenyl carbon atoms remains. The heteroalkenyl group may be C-linked or hetero-linked, *i.e.* it may be linked to the remainder of the molecule through a carbon atom or through O, S(O)t or N. Heteralkenyl may be substituted.

The term "heterocycloalkenyl" includes monovalent cycloalkenyl groups in which up to three carbon atoms, or up to two carbon atoms, or one carbon atom, are each replaced independently by O, S(O), or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, *i.e.* it may be linked to the remainder of the molecule through a carbon atom or through a nitrogen atom. Heterocycloalkenyl may be substituted.

The term "heterocycloalkenylene" includes divalent cycloalkenylene groups in which up to three carbon atoms, or up to two carbon atoms, or one carbon atom, are each replaced independently by O, S(O), or N, provided at least one of the cycloalkenylene carbon atoms remains. The heterocycloalkenylene group may be C-linked or N-linked, *i.e.* it may be linked to the remainder of the molecule through a carbon atom or through a nitrogen atom. Heterocycloalkenylene may be substituted.

The term "heteroalkynyl" includes monovalent alkynyl groups, for example, C₁₋₆₅alkynyl groups, C₁₋₁₇alkynyl groups or C₁₋₁₀alkynyl groups, in which up to twenty carbon atoms, or in which up to ten carbon atoms, or up to two carbon atoms, or one carbon atom, are each replaced independently by O, S(O), or N, provided at least one of the alkynyl carbon atoms remains. The heteroalkynyl group may be C-linked or hetero-linked, *i.e.* it may be linked to the remainder of the molecule through a carbon atom or through O, S(O)t or N. Heteroalkynyl may be substituted.

The term "heteroalkylene" includes divalent alkylene groups, for example, C₁₋₆₅alkylene groups, C₁₋₁₇alkylene groups or C₁₋₁₀alkylene groups, in which up to twenty carbon atoms, or in which up to ten carbon atoms, or up to two carbon atoms, or one carbon atom, are each replaced independently by O, S(O), or N, provided at least one of the alkylene carbon atoms remains. Heteroalkynylene may be substituted.

The term "heteroalkenylene" includes divalent alkenylene groups, for example, C₁₋₆₅alkenylene groups, C₁₋₁₇alkenylene groups or C₁₋₁₀alkenylene groups, in which up to twenty carbon atoms, or in which up to ten carbon atoms, or up to two carbon atoms, or one carbon atom, are each replaced independently by O, S(O), or N, provided at least one of the alkenylene carbon atoms remains. Heteroalkenylene may be substituted.

The term "aryl" includes monovalent, aromatic, cyclic hydrocarbyl groups, such as phenyl or naphthyl (e.g. 1-naphthyl or 2-naphthyl). In general, the aryl groups may be monocyclic or polycyclic fused ring aromatic groups. Particular aryl are C₆-C₁₄aryl. Aryl may be substituted.

Other examples of aryl groups are monovalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

The term "arylalkyl" means alkyl substituted with an aryl group, e.g. benzyl.

The term "arylene" means a divalent, aromatic, cyclic hydrocarbyl groups, such as phenylene or naphthylene (e.g. 1-naphthylene or 2-naphthylene). In general, the arylene groups may be monocyclic or polycyclic fused ring aromatic groups. Particular arylenes are C₆-C₁₄arylene. Arylene may be substituted.

The term "heteroaryl" includes monovalent aryl groups in which one or more carbon atoms are each replaced by heteroatoms independently selected from O, S, N, and NR^{N}, where R^{N} is defined below (and in one embodiment is H or alkyl (e.g. C₁₋₆alkyl)). Heteroaryl may be substituted.

In general, the heteroaryl groups may be monocyclic or polycyclic (e.g. bicyclic) fused ring heteroaromatic groups. Typically, heteroaryl groups contain 5-14 ring members (particularly 5-10 members) wherein 1, 2, 3 or 4 ring members are independently selected from O, S, N, and NR^{N}. A heteroaryl group is suitably a 5, 6, 9 or 10 membered, e.g. 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members wherein 1, 2, 3 or 4 ring members are independently selected from O, S, N, and NR^{N}.

5-Membered monocyclic heteroaryl groups may contain 1 ring member which is an -NR^{N}-group, an -O- atom or an -S- atom and, optionally, 1-3 ring members (e.g. 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

6-Membered monocyclic heteroaryl groups may contain 1 or 2 ring members which are =N- atoms (where the remainder of the 6 ring members are carbon atoms).

Bicyclic heteroaromatic groups include fused-ring heteroaromatic groups containing 9-14 ring members wherein 1, 2, 3, 4 or more ring members are independently selected from O, S, N, and NR^{N}.

9-Membered bicyclic heteroaryl groups may contain 1 ring member which is an -NR^{N}-group, an-O- atom or an -S- atom and, optionally, 1-3 ring members (e.g. 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

10-Membered bicyclic heteroaryl groups may contain 1-3 ring members which are =N-atoms (where the remainder of the 10 ring members are carbon atoms).

Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylene" means divalent aryl groups in which one or more carbon atoms are each replaced by heteroatoms independently selected from O, S, N, and NR^{N}, where R^{N} is defined below (and in one embodiment is H or alkyl (e.g. C₁₋₆alkyl)). Heteroarylene may be substituted.

The term "heteroarylalkyl" means alkyl substituted with a heteroaryl group.

Examples of acyl groups include alkyl-C(=O)-, cycloalkyl-C(=O)-, alkenyl-C(=O)-, cycloalkenyl-C(=O)-, heteroalkyl-C(=O)-, heterocycloalkyl-C(=O)-, aryl-C(=O)- or heteroaryl-C(=O)-, in particular, alkyl-C(=O)- and aryl-C(=O)-.

Unless indicated explicitly otherwise, where combinations of groups are referred to herein as one moiety, e.g. arylalkyl, the last mentioned group contains the atom by which the moiety is attached to the rest of the molecule.

The term thiohydroxyl or thiol, as used herein, refers to a group of the formula -SH.

Where reference is made to a carbon atom of an alkyl group or other group being replaced by O, S(O), or N, what is intended is that: 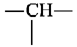
is replaced by 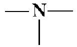
-CH= is replaced by -N=;
=C-H is replaced by =N; or
-CH₂- is replaced by -O-, -S(O)t- or -NR^{N}-.

By way of clarification, in relation to the above mentioned heteroatom containing groups (such as heteroalkyl *etc.),* where a numerical of carbon atoms is given, for instance C₃₋₆heteroalkyl, what is intended is a group based on C₃₋₆alkyl in which one of more of the 3-6 chain carbon atoms is replaced by O, S(O)t or N. Accordingly, a C₃₋₆heteroalkyl group, for example, will contain less than 3-6 chain carbon atoms.

Where mentioned above, R^{N} is H, alkyl, cycloalkyl, aryl, heteroaryl, -C(O)-alkyl, -C(O)-aryl, -C(O)-heteroaryl, -S(O)t-alkyl, -S(O)t-aryl or -S(O),-heteroaryl. R^{N} may, in particular, be H, alkyl (e.g. C₁₋₆alkyl) or cycloalkyl (e.g. C₃₋₆cycloalkyl).

Where mentioned above, t is independently 0, 1 or 2, for example 2. Typically, t is 0.

Where a group has at least 2 positions which may be substituted, the group may be substituted by both ends of an alkylene or heteroalkylene chain to form a cyclic moiety.

Optionally substituted groups (e.g. alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene, aryl, arylalkyl, arylheteroalkyl, heteroaryl, heteroarylalkyl or heteroarylheteroalkyl groups *etc.)* may be substituted or unsubstituted or may be unsubstituted. Typically, substitution involves the notional replacement of a hydrogen atom with a substituent group, or two hydrogen atoms in the case of substitution by =O.

Where substituted, there will generally be 1 to 3 substituents, or 1 or 2 substituents, or 1 substituent.

The optional substituent(s) is/are independently halogen, trihalomethyl, trihaloethyl,-OH, -NH₂, -NO₂, -CN, -N⁺(C₁₋₆alkyl)₂O⁻, -CO₂H, -CO₂C₁₋₆alkyl, -SO₃H, -SOC₁₋₆alkyl, -SO₂C₁₋₆alkyl, -SO₃C₁₋₆alkyl, -OC(=O)OC₁₋₆alkyl, -C(=O)H, -C(=O)C₁₋₆alkyl, -OC(=O)C₁₋₆alky), =O, -NH(C₁₋₆alkyl), -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=0)C₁₋₆alkyl, -C(=S)N(C₁₋₆alkyl)₂, -N(C₁₋₆alkyl)C(=S)C₁₋₆alkyl, -SO₂N(Ci.₆alkyl)₂, -N(C₁₋₆alkyl)S0₂C₁₋₆alkyl, -N(C₁₋₆alkyl)C(=S)N(C₁₋₆alkyl)₂, -N(C₁₋₆alkyl)SO₂N(C₁₋₆alkyl)₂, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalk enyl, -C₃₋₆cycloalkenyl, -C₃₋₆heterocycloalkenyl, -C₂₋₆alkynyl, -C₂₋₆heteroalkynyl, -Z^{u}-C₁₋₆alkyl,-Z^{U}- C₃₋₆cycloalkyl, -Z^{u}-C₂₋₆alkenyl, -Z^{u}-C₃₋₆cycloalkenyl or -Z^{u}-C₂₋₆alkynyl, wherein Z^{u} is independently O, S, NH or N(C₁₋₆alkyl).

In one embodiment, optionally in combination with any of the embodiments provided above or below, the optional substituent(s) is/are independently halogen, trihalomethyl, trihaloethyl, -NO₂, -CN, -N⁺(C₁₋₆alkyl)₂O⁻, -CO₂H, -SO₃H, -SOC₁₋₆alkyl, -SO₂C₁₋₆alkyl, -C(=O)H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or-z^{u}-C₃₋₆cycloalkyl, wherein Z^{u} is defined above. More particularly, the optional substituent(s) is/are independently halogen, trihalomethyl, -NOz, -CN, -CO₂H, -C(=O)C₁₋₆alkyl, =O, -N(C₁₋₆alkyl)₂, -C(=O)NH₂, -C₁₋₆alkyl, -C₃₋₆cycloalkyl, -C_{3- 6}heterocycloalkyl, -Z^{u}C₁₋₆alkyl or -Z^{u}-C₃₋₆cycloalkyl, wherein Z^{u} is defined above. Even more particularly, the optional substituent(s) is/are independently halogen, -NOz, -CN, -CO₂H, =O, -N(C₁₋₆alkyl)₂, -C₁₋₆alkyl, -C₃₋₆cycloalkyl or -C₃₋₆heterocycloalkyl.

In another embodiment, optionally in combination with any of the embodiments provided above or below, the optional substituent(s) is/are independently halogen, -OH, NH₂, NH(C₁₋₆alkyl), -N(C₁₋₆alkyl)₂, -C₁₋₆alkyl, -C₃₋₆cycloalkyl or-C₃₋₆heterocycloalkyl.

The term "polyalkylene glycol" (PAG) refers to compounds having the general formula H-[O-C_{y}H_{2y}]ₓ-OH, such as H-[O-CH_{z}-CH₂]ₓ-OH (polyethylene glycol or PEG) and H-[O-CH(CH₃)-CH₂]ₓ-OH (polypropylene glycol). When found in a compound of the invention the PAG is bound by the bond between a carbon atom and one of the terminal hydroxyl groups e.g. in the case of PEG the substituent would be H-[O-CH₂-CH₂]ₓ-. The polyalkylene glycols used in the compounds of the invention, unless otherwise defined, may have a molecular weight of from 500 to 20000 g/mol, particularly from 1000 to 10000 g/mol, more particularly from 2000 to 5000 g/mol, more particularly from 2000 to 3500 g/mol. When present, the polyalkylene glycol is either bound directly to the nitrogen atom to which R₃/R₃'/R₃" is attached or bound to the nitrogen atom to which R₃/R₃'/R₃"is attached via a linker moiety, wherein said linker moiety is an alkylene, cycloalkylene, alkenylene, cycloalkenylene, heteroalkylene, heterocycloalkylene, arylene or heteroarylene group.

### Salts and polymorhs

For the purposes of the present invention the term "polymer" includes pharmaceutically acceptable derivatives thereof and polymorphs, isomers and isotopically labelled variants thereof. The term "pharmaceutically acceptable derivative" of a polymer includes any pharmaceutically acceptable salt, solvate, hydrate or prodrug of the polymer.

The term "pharmaceutical acceptable salt" as used herein refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutical acceptable salts are well known in the art.

Examples of pharmaceutical acceptable salts include nontoxic acid addition salts, that is salts formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid by using other methods used in the art such as ion exchange. Other pharmaceutical acceptable salts include adipate, alginate, ascorbate, aspartate, benzenesulfonate, benzoate, bisulphate, borate, butyrate, camphorate, camphorsulfonate, citrate, cyclopentanepropionate, digluconate, dodecylsulphate, ethanesulfonate, formate, fumarate, glucoheptonate, glycerophosphate, gluconate, hemisulphate, heptanoate, hexanoate, hydroiodide, 2-hydroxy-ethanesulfonate, lactobionate, lactate, laurate, lauryl sulphate, malate, maleate, malonate, methanesulfonate, 2-naphthalenesulfonate, nicotinate, nitrate, oleate, oxalate, palmitate, pamoate, pectinate, persulphate, 3-phenylpropionate, phosphate, picrate, pivalate, propionate, stearate, succinate, sulphate, tartrate, thiocyanate, p-toluenesulfonate, undecanoate, valerate salts, and the like. Salts derived from appropriate bases include alkali metal, alkaline earth metal, and ammonium. Representative alkali or alkaline earth metal salts include sodium, lithium, potassium, calcium, magnesium, and the like. Further pharmaceutical acceptable salts include, when appropriate, nontoxic ammonium, quaternary ammonium, and amine cations formed using counterions such as halide, hydroxide, carboxylate, sulphate, phosphate, nitrate, lower alkyl sulfonate and aryl sulfonate.

Pharmaceutically acceptable basic salts of the polymers may include, but are not limited to, metal salts such as alkali metal or alkaline earth metal salts (e.g. sodium, potassium, magnesium or calcium salts) and zinc or aluminium salts. Pharmaceutically acceptable basic salts of the polymers as defined herein may include, but are not limited to, salts formed with ammonia or pharmaceutically acceptable organic amines or heterocyclic bases such as ethanolamines (e.g. diethanolamine), benzylamines, N-methyl-glucamine, amino acids (e.g. lysine) or pyridine.

Hemisalts of acids and bases may also be formed, e.g. hemisulphate salts.

Pharmaceutically acceptable salts of the polymers of the invention may be prepared by methods well-known in the art.

The polymers as defined herein may exist in both unsolvated and solvated forms. The term "solvate" includes molecular complexes comprising the polymer and one or more pharmaceutically acceptable solvent molecules such as water or C₁₋₆ alcohols, e.g. ethanol. The term "hydrate" means a "solvate" where the solvent is water.

The polymers may exist in solid states from amorphous through to crystalline forms. All such solid forms are included within the invention.

The polymers may exist in one or more geometrical, optical, enantiomeric, diastereomeric and tautomeric forms, including but not limited to *cis-* and trans-forms, *E-* and Z-forms, R-, S- and meso-forms, keto- and enol-forms. All such isomeric forms are included within the invention. The isomeric forms may be in isomerically pure or enriched form, as well as in mixtures of isomers (e.g. racemic or diastereomeric mixtures).

The invention includes pharmaceutically acceptable isotopically-labelled polymers wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled polymers of the present invention which incorporate a radioactive isotope are useful in drug and/or substrate tissue distribution studies. The radioactive isotopes ³H and ¹⁴C are particularly useful for this purpose in view of their ease of incorporation and ready means of detection.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled polymers can generally be prepared by conventional techniques known to those skilled in the art or by processes analogous to those described herein using an appropriate isotopically-labelled reagent in place of the non-labelled reagent previously employed.

It will be appreciated that the polymers, as described herein, may be substituted with any number of substituents or functional moieties. The terms substituted, whether preceded by the term "optionally" or not, and substituent, as used herein, refer to the ability, as appreciated by one skilled in this art, to change one functional group for another functional group provided that the valency of all atoms is maintained. When more than one position in any given structure may be substituted with more than one substituent selected from a specified group, the substituent may be either the same or different at every position. The substituents may also be further substituted (e.g., an aryl group substituent may have another substituent off it, such as another aryl group, which is further substituted with fluorine at one or more positions).

### Polymers of the invention

As mentioned above, a first aspect of the invention relates to a polymer or a pharmaceutically acceptable salt thereof which comprises a first polymer building block and a second polymer building block, wherein the first and second polymer building blocks are covalently bonded through at least a common lateral chain, and the first polymer building block is an OM-pBAE.

For the purposes of the invention, the term "common lateral chain" refers to a lateral chain of the polymer of the invention that bonds covalently the first and the second polymer building blocks. In other words, the common lateral chain is a birradical moiety which covalently attaches the first and second polymer building blocks, such that one end of the each biradical moiety is attached to the first polymer building block at a position different from the ends of the first polymer building block, and the other end of the biradical moiety is attached to the second polymer building block at a position different from the ends of the second polymer building block.

Thus, the invention also relates to a polymer, or a pharmaceutically acceptable salt thereof, which comprises a first polymer building block and a second polymer building block wherein:
a) the first polymer building block is an oligopeptide-modified poly(beta-aminoester), and
b) the first and second polymer building blocks are covalently bonded through at least a biradical moiety, wherein
   - one end of each biradical moiety is attached to the first polymer building block at a position different from the ends of the first polymer building block, and
   - the other end of each biradical moiety is attached to the second polymer building block at a position different from the ends of the second polymer building block.

The term "lateral chain", also referred to as "side chain", or "pendant chain" of a polymer, refers to a covalently bonded chain of atoms which is covalently bonded to the polymer main chain and the chain length of which is shorter than that of the polymer main chain. The term "main chain", also referred to as "backbone", "primary chain", or "central chain" of a polymer refers to the longest covalently bonded chain of atoms of the polymer.

In one embodiment, optionally in combination with any of the embodiments provided above or below, the common lateral chain is attached at one end to a nitrogen atom of the main chain of the first polymer building block, and at the other end to a nitrogen atom of the main chain of the second polymer building block.

In another embodiment, optionally in combination with any of the embodiments provided above or below, the common lateral chain is a biradical of formula (vii) 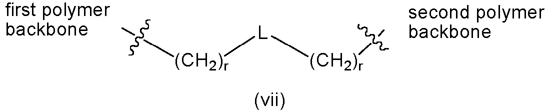 wherein:
L is -C(=O)-Z-, -NH-C(=O)-Z-, -O-C(=O)-Z-, -C(=O)-O-Z-, -C(=O)-NH-Z, -(OCH₂CH₂)ₛC(=O)-Z-, -(OCH₂CH₂)ₛNH-C(=O)-Z-, -(OCH₂CH₂)ₛO-C(=O)-Z-, -(OCH₂CH₂)ₛC(=O)-O-Z-, and -(OCH₂CH₂)ₛC(=O)-NH-Z-; Z is a heterocycloalkenylene comprising at least two nitrogen atoms and two fused rings; and each r is independently an integer from 1 to 10. Heterocycloalkenylene may be optionally substituted.

More particularly, the common lateral chain is a biradical of formula (vii) attached at one end to a nitrogen atom of the main chain of the first polymer building block, and at the other end to a nitrogen atom of the main chain of the second polymer building block.

In a more particular embodiment, optionally in combination with any of the embodiments provided above or below, the common lateral chain is a biradical of formula (vii), wherein Z is a biradical selected from the group consisting of formulas (viii₁) to (viii₄): 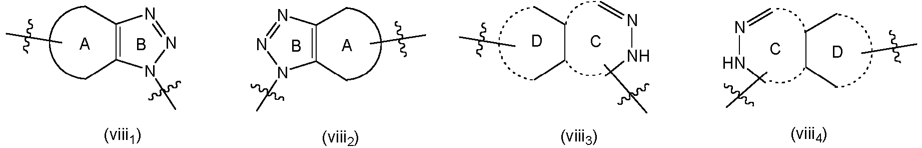
wherein A is a 6 to 14-membered carbocyclic or heterocyclic ring comprising from 1 to 3 rings, wherein the ring members are selected from C, CH, O, S, N, and NH; B is a triazole ring; C is a 6-membered heterocyclic ring comprising two nitrogen atoms wherein the remaining ring members are selected from C and CH; D is a 3 to 10-membered carbocyclic ring comprising 1 or 2 rings, wherein the ring members are selected from C, and CH;
wherein when Z is a biradical of formula (viii₁) it is attached at one end to a nitrogen atom of the main chain of the first polymer building block through ring A, and at the other end to a nitrogen atom of the main chain of the second polymer building block through ring B;
when Z is a biradical of formula (viii₂) it is attached at one end to a nitrogen atom of the main chain of the first polymer building block through ring B, and at the other end to a nitrogen atom of the main chain of the second polymer building block through ring A;
when Z is a biradical of formula (viii₃) it is attached at one end to a nitrogen atom of the main chain of the first polymer building block through ring C, and at the other end to a nitrogen atom of the main chain of the second polymer building block through ring D;
when Z is a biradical of formula (viii₄) it is attached at one end to a nitrogen atom of the main chain of the first polymer building block through ring D, and at the other end to a nitrogen atom of the main chain of the second polymer building block through ring C.

Rings A, C, and D may be optionally substituted.

In an even more particular embodiment, optionally in combination with any of the embodiments provided above or below, the common lateral chain is a biradical of formula (vii), wherein Z is a biradical selected from the group consisting of formulas (viii₅) to (viii₁₁):

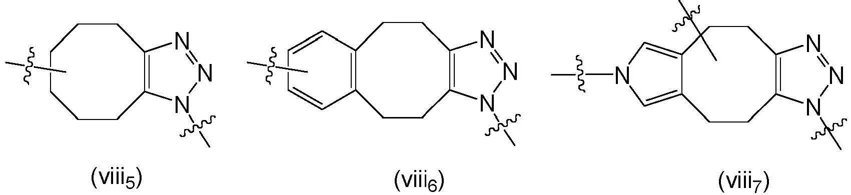

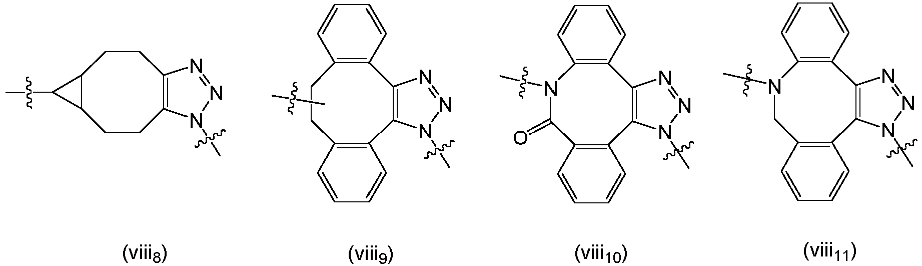

Any of the rings of the biradicals (viii₅) to (viii₁₁) may be optionally substituted when chemically possible.

In an even more particular embodiment, optionally in combination with any of the embodiments provided above or below, the common lateral chain is a biradical of formula (vii), wherein each r is independently an integer from 2 to 5, L is a birradical of formula -C(=O)-Z-, and Z is a biradical selected from the group consisting of formulas (viii₅) to (viii₁₁).

In another embodiment, optionally in combination with any of the embodiments provided above or below, the first polymer building block and the second polymer building block have a molecular weight of from 500 to 20000 g/mol, particularly from 1000 to 10000 g/mol, more particularly from 2000 to 5000 g/mol, even more particularly from 2000 to 3500 g/mol, and even more particularly from 2000 to 3000 g/mol.

The molecular weight refers to the average molecular weight (MW) and can be measured by methods well-known in the art such as size exclusion chromatography (SEC) (also referred to as gel permeation chromatography (GPC)) matrix assisted laser desorption ionization-time-of-flight mass spectrometry (MALDI-TOF MS). The composition and ratios of the polymers are characterized by 1H-NMR spectroscopy among other techniques.

In another embodiment, optionally in combination with any of the embodiments provided above or below, the invention relates to a polymer as defined in the first aspect, wherein the first polymer building block is of formula (I), more particularly of formula (Ia).

In another embodiment, optionally in combination with any of the embodiments provided above or below, the invention relates to a polymer as defined in the first aspect, wherein the second polymer building block is of formula (II), more particularly of formula (Ila).

In another embodiment, optionally in combination with any of the embodiments provided above or below, the invention relates to a polymer as defined in the first aspect wherein the second polymer building block is of formula (II), more particularly of formula (IIb).

In another embodiment, optionally in combination with any of the embodiments provided above or below, the invention relates to a polymer as defined in the first aspect, wherein the first polymer building block is of formula (I), more particularly of formula (Ia), and the second polymer building block is of formula (II), more particularly of formula (IIa). More particularly, in this embodiment, the common lateral chain is a biradical of formula (vii), wherein more particularly Z is a biradical selected from the group consisting of formulas (viii₁) to (viii₄), even more particularly Z is a biradical selected from the group consisting of formulas (viii₅) to (viii₁₁).

In another embodiment, optionally in combination with any of the embodiments provided above or below, the invention relates to a polymer as defined in the first aspect, wherein the first polymer building block is of formula (I), more particularly of formula (Ia); the second polymer building block is of formula (II), more particularly of formula (IIb). More particularly, in this embodiment, the common lateral chain is a biradical of formula (vii), wherein more particularly Z is a biradical selected from the group consisting of formulas (viii₁) to (viii₄), even more particularly Z is a biradical selected from the group consisting of formulas (viii₅) to (viii₁₁).

### First polymer building block

In another embodiment, optionally in combination with any of the embodiments provided above or below, the first polymer building block is an oligopeptide-modified poly(beta-aminoester) of formula (I): 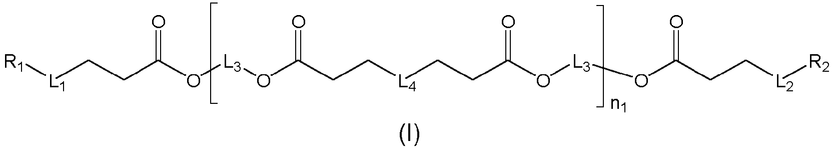 wherein:
each L₃ is independently selected from the group consisting of alkylene, alkenylene, heteroalkylene, heteroalkenylene, arylene, and heteroarylene; or alternatively
at least one occurrence of L₃ is a biradical of formula (i₁): 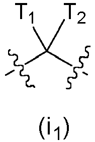 wherein:
   T₁ is a radical of formula (ii₁): 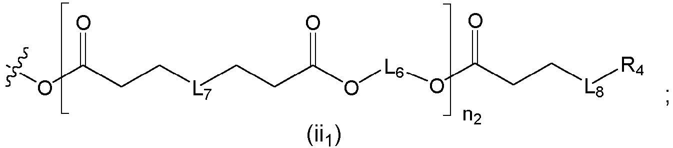
   T₂ is selected from the group consisting of H, alkyl, and a radical of formula (ii₁);
and the remaining L₃ groups are independently selected at each occurrence from the group consisting of alkylene, alkenylene, heteroalkylene, heteroalkenylene, arylene, and heteroarylene;
each L₄ and L₇ are independently selected from the group consisting of a biradical of formula (iii₁) and formula (iv₁): 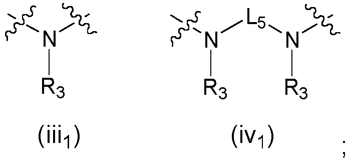
each L₁, L₂, and L₈ are independently selected from the group consisting of O, S, NRₓ, a bond, and a biradical of formula (v₁); 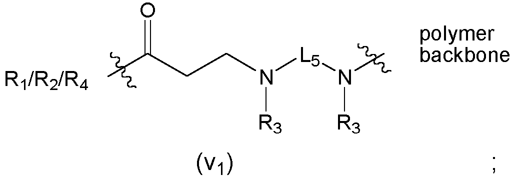
each L₅ and L₆ are independently selected from the group consisting of alkylene, alkenylene, heteroalkylene, heteroalkenylene, arylene, and heteroarylene;
R₁, R₂ and each R₄ are independently selected from the group consisting of an oligopeptide and R_{y}; wherein at least one of R₁, R₂ and R₄ is an oligopeptide;
each R₃ is independently selected from the group consisting of a bond, hydrogen, halogen, alkyl, cycloalkyl, alkenyl, cycloalkenyl, heteroalkyl, heterocycloalkyl, acyl, aryl, heteroaryl, a polyalkylene glycol, -(CH_{z})ₚOH, -(CH₂)₂(OCH₂CH₂)_{q}OH, -(CH₂)ₚNH₂, and -(CH₂)₂(OCH₂CH₂)_{q}NH₂;
each Rₓ and R_{y} is independently selected from the group consisting of hydrogen, halogen, alkyl, cycloalkyl, alkenyl, cycloalkenyl, heteroalkyl, heterocycloalkyl, acyl, aryl, and heteroaryl; and
n₁ and n₂ are independently an integer from 5 to 1000; p is an integer from 1 to 20; and q, is an integer from 1 to 10,
provided that at least one R₃ in any of L₁, L₂, L₃, L₄, L₇ and L₈ is a bond which binds the first building block to one of the ends of the at least one common lateral chain.

It is understood that L₁, L₂ and L₈ are biradicals, and thus, biradical L₁ connects with R₁ at one end and with the polymer chain at the other end, biradical L₂ connects with R₂ at one end and with the polymer chain at the other end, and biradical L₈ connects with R₄ at one end and with the polymer chain at the other end. Thus, it is understood that the "R₁/R₂/R₄" and "polymer chain" are depicted in the structure above to establish the orientation of the structure of L₁, L₂ and L₃, i.e., to specify which end connects with R₁, R₂ or R₄ and which end connects with the polymer chain. The same is applicable to L₁', L₂'and L₈' in the polymer building block of formula (II).

In the compounds disclosed herein above and below, where a repeating unit is depicted (by square brackets), each group (e.g., L₃, L₄) within the square brackets is independently selected from the provided definitions for each single repeating unit. In other words, the repeating units within a particular polymer need not be identical.

In one embodiment, optionally in combination with any of the embodiments provided above or below, in the polymer building block of formula (I), n₁ and n₂ are independently from 10 to 700, more particularly from 20 to 500.

In another embodiment, optionally in combination with any of the embodiments provided above or below, the molecular weight of the polymer building block of formula (I) is from 500 to 150000 g/mol, more particularly from 700 to 100000 g/mol, even more particularly from 2000 to 50000 g/mol, and even more particularly from 2000 to 40000 g/mol.

In another embodiment, optionally in combination with any of the embodiments provided above or below, in the polymer building block of formula (I), L₃ and L₅ are independently selected from alkylene, alkenylene, heteroalkylene or heteroalkenylene and including polyethylene glycol linkers. Said alkylene, alkenylene, heteroalkylene or heteroalkenylene moieties may be of 1-20 carbon atoms, particularly of 1-12 carbon atoms, more particularly of 1-6 carbon atoms. Said polyethylene glycol linkers may be of 3 to 25 atoms in length, particularly of 3 to 18 atoms in length. More particularly, L₃ and L₅ are independently selected from alkylene moieties, particularly of 1-12 carbon atoms, more particularly of 1-6 carbon atoms, more particularly of 3-5 carbon atoms, and in a particular embodiment of 4 carbon atoms. In a particular embodiment, L₃ and L₃' are selected from -CH₂-, -(CH₂)₂-, -(CH₂)₃-, -(CH₂)₄-, -(CH₂)₅-, and -(CH₂)₆-.

In another embodiment, optionally in combination with any of the embodiments provided above or below, in the polymer building block of formula (I), each L₃ is independently selected from the group consisting of alkylene, alkenylene, heteroalkylene, heteroalkenylene, arylene, and heteroarylene; particularly L₃ represents alkylene.

In another embodiment, optionally in combination with any of the embodiments provided above or below, in the polymer building block of formula (I), each L₄ is a biradical of formula (iii₁). More particularly, the polymer building block of formula (I) comprises at least two different L₄ biradicals of formula (iii₁).

In another embodiment, optionally in combination with any of the embodiments provided above or below, in the polymer building block of formula (I), each R₃ is independently selected from the group consisting of a bond, alkyl, -(CH_{z})ₚOH, -(CH₂)₂(OCH₂CH₂)_{q}OH, -(CH₂)ₚNH₂, and -(CH₂)₂(OCH₂CH₂)_{q}NH₂, wherein p is an integer from 1 to 20, and q is an integer from 1 to 10, more particularly p and q are independently an integer from 1 to 10; provided that at least one R₃ in any of L₁, L₂, L₃, L₄, L₇ and L₈ is a bond which binds the first building block to one of the ends of the at least one common lateral chain.

In another embodiment, optionally in combination with any of the embodiments provided above or below, in the polymer building block of formula (I), at least one R₃ is alkyl and at least one R₃ is -(CH_{z})ₚOH, wherein p is an integer from 1 to 10.

In another embodiment, optionally in combination with any of the embodiments provided above or below, in the polymer building block of formula (I), L₁ and L₂ are a bond.

The expression "L₁ represent a bond" means that the polymer building block of formula (I) is one wherein:

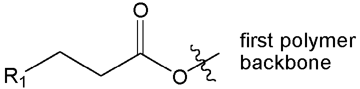

The expression "L₂ represent a bond" means that the polymer building block of formula (I) is one wherein:

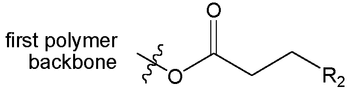

The same is applicable to L₁' and L₂' in the polymer building block of formula (II) or to L₁" and L₂" in the polymer of formula (III).

In the polymer building block of formula (I) at least one of R₁, R₂ and R₄ is an oligopeptide. In one embodiment, optionally in combination with any of the embodiments provided above or below, in the polymer building block of formula (I), one of R₁, R₂ and R₄ is an oligopeptide and the remaining radicals of R₁, R₂ and R₄ are independently R_{y}. In another embodiment, optionally in combination with any of the embodiments provided above or below, in the polymer building block of formula (I), two of R₁, R₂ and R₄ are independently an oligopeptide and the remaining radical of R₁, R₂ and R₄ is R_{y}. In another embodiment, optionally in combination with any of the embodiments provided above or below, in the polymer building block of formula (I), R₁, R₂ and R₄ are independently an oligopeptide.

In another embodiment, optionally in combination with any of the embodiments provided above or below, in the polymer building block of formula (I), R_{y} is selected from the group consisting of hydrogen, -(CH₂)ₘNH₂, -(CH₂)ₘNHMe, -(CH₂)ₘOH, -(CH₂)ₘCH₃, -(CH₂)₂(OCH₂CH₂)ₘNH₂, -(CH₂)₂(OCH₂CH₂)ₘOH, and -(CH₂)₂(OCH₂CH₂)ₘCH₃ wherein m is an integer from 1 to 20, for example from 1 to 5. Particularly, R_{y} is selected from the group consisting of-(CH₂)ₘNH₂, -(CH₂)ₘNHMe, and-(CH₂)₂(OCH₂CH₂)ₘNH₂.

In another embodiment, optionally in combination with any of the embodiments provided above or below, in the polymer building block of formula (I), R₁ and R₂ are independently an oligopeptide, more particularly R₁ and R₂ are the same oligopeptide, more particularly, R₁ and R₂ are the same positively charged oligopeptide at pH 7, even more particularly R₁ and R₂ are an oligopeptide of formula of formula (vi): 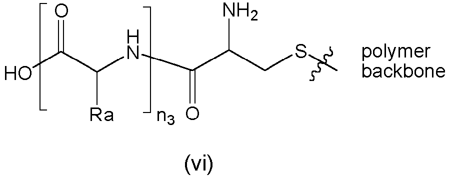 wherein n₃ is an integer from 2 to 19, and each Rₐ is selected from the group consisting of H₂NC(=NH)-NH(CH₂)₃-, H₂N(CH₂)₄-, and (1*H*-imidazol-4-yl)-CH₂-.

In another embodiment, optionally in combination with any of the embodiments provided above or below, the polymer building block of formula (I) is a polymer building block of formula (Ia), wherein:
- each L₃ is independently selected from the group consisting of alkylene, alkenylene, heteroalkylene, heteroalkenylene, arylene, and heteroarylene; particularly L₃ represents alkylene;
- each L₄ is a biradical of formula (iii₁);
- each R₃ is independently selected from the group consisting of a bond,
   alkyl, -(CH_{z})ₚOH, -(CH₂)₂(OCH₂CH₂)_{q}OH, -(CH₂)ₚNH₂, and -(CH₂)₂(OCH₂CH₂)_{q}NH₂, wherein p is an integer from 1 to 20, and q is an integer from 1 to 10, provided that at least one R₃ in any of L₁, L₂, L₃, L₄, L₇ and L₈ is a bond which binds the first building block to one of the ends of the at least one common lateral chain;
   L₁ and L₂ are a bond;
- R₁ and R₂ are independently an oligopeptide, more particularly R₁ and R₂ are the same oligopeptide, more particularly, R₁ and R₂ are the same positively charged oligopeptide at pH 7, even more particularly R₁ and R₂ are an oligopeptide of formula of formula (vi).

In another embodiment, optionally in combination with any of the embodiments provided above or below, the polymer building block of formula (Ia) comprises at least two different L₄ biradicals of formula (iii₁), more particularly wherein at least one R₃ is alkyl, and at least one R₃ is -(CH₂)ₚOH.

### Second polymer building block

In one embodiment, optionally in combination with any of the embodiments provided above or below, the second polymer building block is functionalized with one or more active ingredients, cell-targeting ligands, diagnostic agents, imaging agents or combinations thereof; more particularly the second polymer building block is functionalized with one or more cell-targeting ligands.

In a more embodiment, optionally in combination with any of the embodiments provided above or below, the second polymer building block comprises one or more moieties selected from the group consisting of active ingredient moieties, cell-targeting moieties, diagnostic agent moieties, imaging agent moieties and combinations thereof, wherein each moiety is selected from the group consisting of R₅, -NH-(CH₂)ₚ-R₅, -O-(CH₂)ₚ-R₅, - NH-(CH₂CH₂)ₛ-(OCH₂CH₂)_{q}-R₅, -O-(CH₂CH₂)ₛ-(OCH₂CH₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is an active ingredient, a cell-targeting ligand, a diagnostic agent, or an imaging agent; more particularly R₅ is a cell-targeting ligand; p is an integer from 1 to 20; and q, is an integer from 1 to 10.

In another embodiment, optionally in combination with any of the embodiments provided above or below, in the polymer of the invention the second polymer building block is selected from the group consisting of a poly(beta-aminoester); a polyalkylene glycol, such as polyethylene glycol (PEG) or poly(ethylene oxide) (PEO); a polyester such as polylactic acid (PLA), polyglycolic acid (PGA), or polylactic acid-co-glycolic acid (PLGA); a polyacrylate such as poly(N-isopropylacrylamide) (PNIPAM), poly(ethylene glycol)-block-poly(2-(Dimethylamino)ethyl methacrylate) (pDMAEMA), poly(methyl methacrylate) (PMMA), or poly(2-hydroxyethyl methacrylate) PHEMA, or polyacrylic acid (PAA); a halogen-containing polymer such as polyvinyl chloride (PVC); a poly(4-N-Bocvinylaniline) (PBVA); or polystyrene (PS). More particularly, the second polymer building block is a poly(beta-aminoester) or a polyalkylene glycol, even more particularly the second polymer building block is a poly(beta-aminoester).

In another embodiment, optionally in combination with any of the embodiments provided above or below, in the polymer of the invention the second polymer building block is a poly(beta-aminoester) of formula (II): 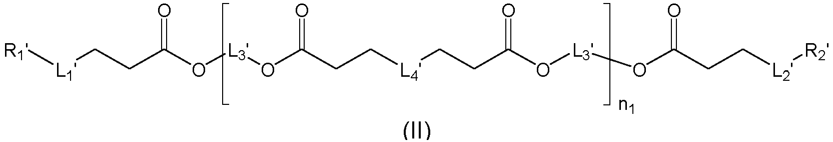 wherein:
each L₃' is independently selected from the group consisting of alkylene, alkenylene, heteroalkylene, heteroalkenylene, arylene, and heteroarylene; or alternatively
at least one occurrence of L₃' is a biradical of formula (i₁'): 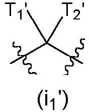 wherein:
   T₁' is a radical of formula (ii₁'): 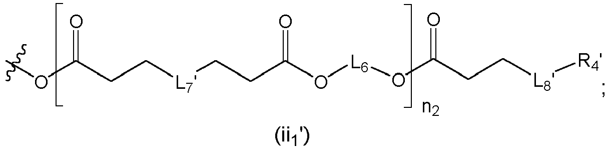
   T₂' is selected from the group consisting of H, alkyl and a radical of formula (ii₁');
and the remaining L₃' groups are independently selected at each occurrence from the group consisting of alkylene, alkenylene, heteroalkylene, heteroalkenylene, arylene, and heteroarylene;
each L₄' and L₇' are independently selected from the group consisting of a biradical of formula (iii₁') and of formula (iv₁'): 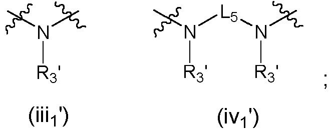
each L₁', L₂', and L₈' are independently selected from the group consisting of O, S, NRₓ, a bond, and a biradical of formula (v₁'); 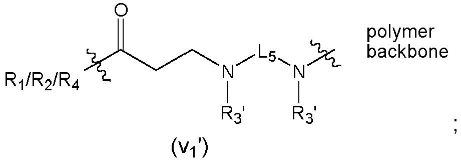
each L₅ and L₆ are independently selected from the group consisting of alkylene, alkenylene, heteroalkylene, heteroalkenylene, arylene, and heteroarylene;
R₁', R₂' and each R₄' are independently selected from the group consisting of an oligopeptide, R_{y}, and a moiety selected from the group consisting of an active ingredient moiety, a cell-targeting moiety, a diagnostic agent moiety, and an imaging agent moiety; more particularly the moiety is a cell-targeting moiety comprising a cell-targeting ligand;
each R₃' is independently selected from the group consisting of a bond, hydrogen, halogen, alkyl, cycloalkyl, alkenyl, cycloalkenyl, heteroalkyl, heterocycloalkyl, acyl, aryl, heteroaryl, a polyalkylene glycol, -(CH_{z})ₚOH, -(CH₂)₂(OCH₂CH₂)_{q}OH, -(CH₂)ₚNH₂, -(CH₂)₂(OCH₂CH₂)_{q}NH₂, and a moiety selected from the group consisting of an active ingredient moiety, a cell-targeting moiety, a diagnostic agent moiety, and an imaging agent moiety; more particularly the moiety is a cell-targeting moiety comprising a cell-targeting ligand;
each Rₓ and R_{y} is independently selected from the group consisting of hydrogen, halogen, alkyl, cycloalkyl, alkenyl, cycloalkenyl, heteroalkyl, heterocycloalkyl, acyl, aryl, and heteroaryl; and
n₁ and n₂ are independently an integer from 5 to 1000; p is an integer from 1 to 20; and q, is an integer from 1 to 10;
provided that the second polymer building block comprises at least an active ingredient, a cell-targeting ligand, a diagnostic agent, or an imaging agent; more particularly at least a cell-targeting ligand; and that at least one R₃' in any of L₁', L₂', L₃', L₄', L₇' and L₈' is a bond which binds the second building block to one of the ends of the at least one common lateral chain.

In one particular embodiment, optionally in combination with any of the embodiments provided above or below, in the polymer building block of formula (II), n₁ and n₂ are independently from 10 to 700, more particularly from 20 to 500.

In another particular embodiment, optionally in combination with any of the embodiments provided above or below, the molecular weight of the polymer building block of formula (II) is from 500 to 150000 g/mol, more particularly from 700 to 100000 g/mol, even more particularly from 2000 to 50000 g/mol, and even more particularly from 5000 to 40000 g/mol.

In another particular embodiment, optionally in combination with any of the embodiments provided above or below, in the polymer building block of formula (II), L₃' and L₅ are independently selected from alkylene, alkenylene, heteroalkylene or heteroalkenylene and including polyethylene glycol linkers. Said alkylene, alkenylene, heteroalkylene or heteroalkenylene moieties may be of 1-20 carbon atoms, particularly of 1-12 carbon atoms, more particularly of 1-6 carbon atoms. Said polyethylene glycol linkers may be of 3 to 25 atoms in length, particularly of 3 to 18 atoms in length. More particularly, L₃' and L₅ are independently selected from alkylene moieties, particularly of 1-12 carbon atoms, more particularly of 1-6 carbon atoms, more particularly of 3-5 carbon atoms, and in a particular embodiment of 4 carbon atoms. In a particularly particular embodiment, L₃ and L₃' are selected from -CH₂-, -(CH₂)₂-, -(CH₂)₃-, -(CH₂)₄-, -(CH₂)₅-, and -(CH₂)₆-.

In another particular embodiment, optionally in combination with any of the embodiments provided above or below, in the polymer building block of formula (II), each L₃' is independently selected from the group consisting of alkylene, alkenylene, heteroalkylene, heteroalkenylene, arylene, and heteroarylene; particularly L₃' represents alkylene.

In another particular embodiment, optionally in combination with any of the embodiments provided above or below, in the polymer building block of formula (II), each L₄' is a biradical of formula (iii₁'). More particularly, the polymer building block of formula (II) comprises at least two different L₄' biradicals of formula (iii₁').

In another particular embodiment, optionally in combination with any of the embodiments provided above or below, in the polymer building block of formula (II), each R₃' is independently selected from the group consisting of a bond, alkyl, -(CH_{z})ₚOH, -(CH₂)₂(OCH₂CH₂)_{q}OH, -(CH₂)ₚNH₂, -(CH₂)₂(OCH₂CH₂)_{q}NH₂, and a moiety selected from the group consisting of an active ingredient moiety, a cell-targeting moiety, a diagnostic agent moiety, and an imaging agent moiety; more particularly the moiety is a cell-targeting moiety; more particularly each R₃' is independently selected from the group consisting of a bond, alkyl, -(CH_{z})ₚOH, and a moiety selected from the group consisting of R₅, -NH-(CH₂)ₚ-R₅, -O-(CH₂)ₚ-R₅, -NH-(CH₂CH₂)ₛ-(OCH₂CH₂)_{q}-R₅, -O-(CH_{2C}H₂)ₛ-(OCH_{2C}H₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is an active ingredient, a cell-targeting ligand, a diagnostic agent, or an imaging agent; more particularly R₅ is a cell-targeting ligand, p is an integer from 1 to 20, and q is an integer from 1 to 10, even more particularly p and q are independently an integer from 1 to 10; provided that at least one R₃' in any of L₁', L₂', L₃', L₄', L₇' and L₈' is a bond which binds the second building block to one of the ends of the at least one common lateral chain.

In another particular embodiment, optionally in combination with any of the embodiments provided above or below, in the polymer building block of formula (II), at least one R₃' is alkyl, at least one R₃' is -(CH_{z})ₚOH, and at least one R₃' is a moiety selected from the group consisting of an active ingredient moiety, a cell-targeting moiety, a diagnostic agent moiety, and an imaging agent moiety; more particularly the moiety is selected from the group consisting of R₅, -NH-(CH₂)ₚ-R₅, -O-(CH₂)ₚ-R₅, -NH-(CH₂CH₂)ₛ-(OCH₂CH₂)_{q}-R₅, -O-(CH₂CH₂)ₛ-(OCH_{2C}H₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is an active ingredient, a cell-targeting ligand, a diagnostic agent, or an imaging agent; more particularly R₅ is a cell-targeting ligand; p is an integer from 1 to 20; and q, is an integer from 1 to 10.

In another particular embodiment, optionally in combination with any of the embodiments provided above or below, in the polymer building block of formula (II), L₁' and L₂' are a bond.

In another particular embodiment, optionally in combination with any of the embodiments provided above or below, in the polymer building block of formula (II), at least one of R₁', R₂' and R₄' is a moiety selected from the group consisting of an active ingredient moiety, a cell-targeting moiety, a diagnostic agent moiety, and an imaging agent moiety; more particularly the moiety is a cell-targeting moiety.

In another particular embodiment, optionally in combination with any of the embodiments provided above or below, in the polymer building block of formula (II), R₁' and R₂' are independently a moiety selected from the group consisting of an active ingredient moiety, a cell-targeting moiety, a diagnostic agent moiety, and an imaging agent moiety; more particularly R₁ and R₂ are independently a moiety selected from the group consisting of R₅, -NH-(CH₂)ₚ-R₅, -O-(CH₂)ₚ-R₅, -NH-(CH₂CH₂)ₛ-(OCH₂CH₂)_{q}-R₅, -O-(CH₂CH₂)ₛ-(OCH_{2C}H₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is an active ingredient, a cell-targeting ligand, a diagnostic agent, or an imaging agent; more particularly R₅ is a cell-targeting ligand; even more particularly R₁' and R₂' are the same cell-targeting moiety; p is an integer from 1 to 20; and q, is an integer from 1 to 10.

In another particular embodiment, optionally in combination with any of the embodiments provided above or below, in the polymer building block of formula (II), one of R₁' and R₂' and R₄' is an oligopeptide and the remaining radicals of R₁' and R₂' and R₄' are independently R_{y}. In another embodiment, optionally in combination with any of the embodiments provided above or below, in the polymer building block of formula (II), two of R₁' and R₂' and R₄' are independently an oligopeptide and the remaining radical of R₁' and R₂' and R₄' is R_{y}. In another embodiment, optionally in combination with any of the embodiments provided above or below, in the polymer building block of formula (II), R₁' and R₂' and R₄' are independently an oligopeptide.

In another particular embodiment, optionally in combination with any of the embodiments provided above or below, in the polymer building block of formula (II), R_{y} is selected from the group consisting of hydrogen, -(CH₂)ₘNH₂, -(CH₂)ₘNHMe, -(CH₂)ₘOH, -(CH₂)ₘCH₃, -(CH₂)₂(OCH₂CH₂)ₘNH₂, -(CH₂)₂(OCH₂CH₂)ₘOH, and -(CH₂)₂(OCH₂CH₂)ₘCH₃ wherein m is an integer from 1 to 20, for example from 1 to 5. Particularly, R_{y} is selected from the group consisting of -(CH₂)ₘNH₂, -(CH₂)ₘNHMe, and -(CH₂)₂(OCH₂CH₂)ₘNH₂.

In another particular embodiment, optionally in combination with any of the embodiments provided above or below, in the polymer building block of formula (II), R₁' and R₂' are independently an oligopeptide, more particularly R₁' and R₂' are the same oligopeptide, more particularly, R₁' and R₂' are the same negatively charged oligopeptide at pH 7, even more particularly R₁' and R₂' are an oligopeptide of formula of formula (vi'): 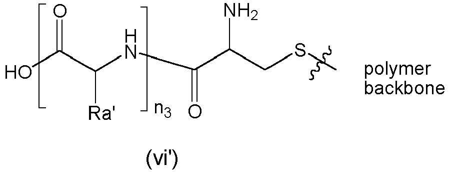 wherein n₃ is an integer from 2 to 19, typically from 3 to 9 or from 3 to 5, and wherein Rₐ' is HO₂C(CH₂)₂- or HO₂C-CH₂-.

In another particular embodiment, optionally in combination with any of the embodiments provided above or below, the polymer building block of formula (II) is a polymer building block of formula (IIa), wherein:
- each L₃' is independently selected from the group consisting of alkylene, alkenylene, heteroalkylene, heteroalkenylene, arylene, and heteroarylene; particularly L₃ represents alkylene;
- each L₄' is a biradical of formula (iii₁');
- each R₃' is independently selected from the group consisting of a bond, alkyl, -(CH_{z})ₚOH, -(CH₂)₂(OCH₂CH₂)_{q}OH, -(CH₂)ₚNH₂, -(CH₂)₂(OCH₂CH₂)_{q}NH₂, and a moiety selected from the group consisting of an active ingredient moiety, a cell-targeting moiety, a diagnostic agent moiety, and an imaging agent moiety; more particularly the moiety is a cell-targeting moiety comprising a cell-targeting ligand; more particularly each R₃' is independently selected from the group consisting of a bond, alkyl, -(CH_{z})ₚOH, and a moiety selected from the group consisting of an active ingredient moiety, a cell-targeting moiety, a diagnostic agent moiety, and an imaging agent moiety; more particularly the moiety is a cell-targeting moiety comprising a cell-targeting ligand; provided that at least one R₃' in any of L₁', L₂', L₃', L₄', L₇' and L₈' is a bond which binds the second building block to one of the ends of the at least one common lateral chain;
- L₁' and L₂' are a bond;
- R₁' and R₂' are independently a moiety selected from the group consisting of an active ingredient moiety, a cell-targeting moiety, a diagnostic agent moiety, and an imaging agent moiety; more particularly R₁' and R₂' are independently a cell-targeting moiety comprising a cell-targeting ligand, even more particularly R₁' and R₂' are the same cell-targeting moiety,

In another particular embodiment, optionally in combination with any of the embodiments provided above or below, the polymer building block of formula (IIa) comprises at least three different L₄' biradicals of formula (iii₁'), more particularly wherein at least one R₃' is alkyl, at least one R₃' is -(CH_{z})ₚOH, and at least one R₃' is a moiety selected from the group consisting of an active ingredient moiety, a cell-targeting moiety, a diagnostic agent moiety, and an imaging agent moiety; more particularly the moiety is a cell-targeting moiety comprising a cell-targeting ligand.

In another particular embodiment, optionally in combination with any of the embodiments provided above or below, in the polymer building block of formula (IIa), the moiety is particularly selected from the group consisting of R₅, -NH-(CH₂)ₚ-R₅, -O-(CH₂)ₚ-R₅, -NH-(CH₂CH₂)ₛ-(OCH₂CH₂)_{q}-R₅, -O-(CH₂CH₂)ₛ-(OCH₂CH₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is an active ingredient, a cell-targeting ligand, a diagnostic agent, or an imaging agent; more particularly R₅ is a cell-targeting ligand; p is an integer from 1 to 20; and q, is an integer from 1 to 10.

In another particular embodiment, optionally in combination with any of the embodiments provided above or below, the polymer building block of formula (II) is a polymer building block of formula (IIb), wherein:
- each L₃' is independently selected from the group consisting of alkylene, alkenylene, heteroalkylene, heteroalkenylene, arylene, and heteroarylene; particularly L₃ represents alkylene;
- each L₄' is a biradical of formula (iii₁');
- each R₃' is independently selected from the group consisting of a bond, alkyl, -(CH_{z})ₚOH, -(CH₂)₂(OCH₂CH₂)_{q}OH, -(CH₂)ₚNH₂, -(CH₂)₂(OCH₂CH₂)_{q}NH₂, and a moiety selected from the group consisting of an active ingredient moiety, a cell-targeting moiety, a diagnostic agent moiety, and an imaging agent moiety; more particularly the moiety is a cell-targeting moiety comprising a cell-targeting ligand; more particularly each R₃' is independently selected from the group consisting of a bond, alkyl, -(CH_{z})ₚOH, and a a moiety selected from the group consisting of an active ingredient moiety, a cell-targeting moiety, a diagnostic agent moiety, and an imaging agent moiety; more particularly the moiety is a cell-targeting moiety comprising a cell-targeting ligand; provided that at least one R₃' in any of L₁', L₂', L₃', L₄', L₇' and L₈' is a bond which binds the second building block to one of the ends of the at least one common lateral chain;
- L₁' and L₂' are a bond;
- R₁' and R₂' are independently an oligopeptide, more particularly R₁' and R₂' are the same oligopeptide, more particularly, R₁' and R₂' are the same negatively charged oligopeptide at pH 7, even more particularly R₁' and R₂' are an oligopeptide of formula of formula (vi'),
provided that the polymer building block of formula (IIb) comprises at least an active ingredient, a cell-targeting ligand, a diagnostic agent, or an imaging agent; more particularly a cell-targeting ligand.

In another particular embodiment, optionally in combination with any of the embodiments provided above or below, the polymer building block of formula (IIb) comprises at least three different L₄' biradicals of formula (iii₁'), more particularly wherein at least one R₃' is alkyl, at least one R₃' is -(CH_{z})ₚOH, and at least one R₃' is moiety selected from the group consisting of an active ingredient moiety, a cell-targeting moiety, a diagnostic agent moiety, and an imaging agent moiety; more particularly the moiety is a cell-targeting moiety comprising a cell-targeting ligand.

In another particular embodiment, optionally in combination with any of the embodiments provided above or below, in the polymer building block of formula (IIa), the moiety is particularly selected from the group consisting of R₅, -NH-(CH₂)ₚ-R₅, -O-(CH₂)ₚ-R₅, -NH-(CH₂CH₂)ₛ-(OCH₂CH₂)_{q}-R₅, -O-(CH₂CH₂)ₛ-(OCH₂CH₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is an active ingredient moiety, a cell-targeting moiety, a diagnostic agent moiety, or an imaging agent moiety; more particularly R₅ is a cell-targeting ligand; p is an integer from 1 to 20; and q, is an integer from 1 to 10.

### Cell-targeting moiety

The second polymer building block includes at least a cell-targeting moiety. In the context of the present invention, the term "cell-targeting moiety" means a moiety that comprises a cell-targeting ligand capable of binding a cell. In certain embodiments, cell-targeting ligands are selective for one or more particular cell type. In certain embodiments, cell-targeting ligands selectively bind a receptor, such as a cell surface receptor.

Illustrative non-limitative examples of cell-targeting ligands include, but are not limited to, an antibody, a receptor ligand, a hormone, a vitamin, and an antigen, however, the present invention is not limited by the nature of the targeting agent. In some embodiments, the cell-targeting ligand is a receptor ligand, such as a ligand for CFTR, EGFR, the estrogen receptor, FGR2, folate receptor, IL-2 receptor, glycoprotein, the retinol binding protein and VEGFR.

In one embodiment, optionally in combination with any of the embodiments provided above or below, the receptor ligand is a carbohydrate receptor (such as a lectin) and the ligand is a sugar (such as mannose or galactose).

In another embodiment, optionally in combination with any of the embodiments provided above or below, the cell-targeting moiety comprises a cell-targeting ligand and linker through which the ligand is attached to the polymer. More particularly, the linker comprises one or more groups selected from among: phosphate, amide, amine, ether, ester, pyrrolidine, disulfide, and methylene.

In another embodiment, optionally in combination with any of the embodiments provided above or below, the cell-targeting moiety is selected from the group consisting of R₅, -NH-(CH₂)ₚ-R₅, -O-(CH₂)ₚ-R₅, -NH-(CH₂CH₂)ₛ-(OCH₂CH₂)_{q}-R₅, -O-(CH₂CH₂)ₛ-(OCH₂CH₂)_{q}-R₅, -NH-(CH₂CH₂)ₛ-(NHCH₂CH₂)_{q}-R₅, and -O-(CH₂CH₂)ₛ-(NHCH₂CH₂)_{q}-R₅, wherein R₅ is a cell-targeting ligand; p is an integer from 1 to 20; and q, is an integer from 1 to 10.

In another embodiment, optionally in combination with any of the embodiments provided above or below, the second polymer building block comprises at least a cell-targeting moiety bonded to a lateral chain of the second polymer building block.

In another embodiment, optionally in combination with any of the embodiments provided above or below, the second polymer building block comprises at least a cell-targeting moiety bonded to one or the two ends of the second polymer building block main chain.

In another embodiment, optionally in combination with any of the embodiments provided above or below, the second polymer building block comprises at least a cell-targeting moiety bonded to a lateral chain of the second polymer building block, and a cell-targeting moiety bonded to one or the two ends of the second polymer building block main chain; more particularly at least a cell-targeting moiety bonded to a lateral chain, and a cell-targeting moiety to each of the two ends of the second polymer building block main chain.

The binding of the cell-targeting moiety to the second polymer building block can be performed following well-known protocols. The skilled person, using their general knowledge, is able to select the most appropriate conditions/reagents, depending on the particular cell-targeting moiety and the particular position of the polymer to which is attached. For example, the cell-targeting moiety can be attached as a lateral chain of the polymer by incorporating it as a monomer when the second polymer building block is synthetized and/or can be incorporated later at a terminal position of the polymer backbone under appropriate conditions.

### Oligopeptides

According to the present invention, an "oligopeptide" comprises a string of at least three amino acids linked together by peptide bonds. Such peptides can contain only natural amino acids, although non-natural amino acids (i.e., compounds that do not occur in nature but that can be incorporated into a polypeptide chain) and/or amino acid analogues as are known in the art may alternatively be employed. Also, one or more of the amino acids in such peptides may be modified, for example, by the addition of a chemical entity such as a carbohydrate group, a phosphate group, a farnesyl group, an isofarnesyl group, a fatty acid group, or a linker for conjugation, functionalization, or other modification, etc.

In one embodiment, optionally in combination with any of the embodiments provided above or below, the oligopeptides in the polymers defined herein typically comprise from 3 to 20 amino acid residues, particularly from 3 to 10 amino acid residues, more particularly from 3 to 6 amino acid residues. Alternatively, the oligopeptides in the polymers defined herein may comprise from 4 to 20 amino acid residues, particularly from 4 to 10 amino acid residues, more particularly from 4 to 6 amino acid residues.

In one embodiment, optionally in combination with any of the embodiments provided above or below, the or each oligopeptide may be hydrophobic. The or each oligopeptide may comprise naturally occurring amino acids that are hydrophobic such as valine, leucine, isoleucine, methionine, tryptophan, phenylalanine, cysteine, tyrosine and alanine; in particular, the or each oligopeptide may comprise valine, leucine, isoleucine, methionine, tryptophan and phenylalanine.

In another embodiment, optionally in combination with any of the embodiments provided above or below, the or each oligopeptide may be hydrophilic. The or each oligopeptide may comprise naturally occurring amino acids that are hydrophilic such as serine, threonine, cysteine, asparagine and glutamine, and may further comprise naturally occurring amino acids that are charged at pH7.

In another embodiment, optionally in combination with any of the embodiments provided above or below, the or each oligopeptide has a net positive charge at pH 7. The or each oligopeptide may comprise naturally occurring amino acids that are positively charged at pH 7, that is, lysine, arginine and histidine. In one embodiment, optionally in combination with any of the embodiments provided above or below, the or each oligopeptide consists of homooligopeptides consisting of naturally occurring amino acids that are positively charged at pH 7, that is, lysine, arginine and/or histidine. For example, the or each oligopeptide may be selected from the group consisting of polylysine, polyarginine, and polyhistidine, each of which may be terminated with cysteine.

In another embodiment, optionally in combination with any of the embodiments provided above or below, the or each oligopeptide is a compound of formula (vi): 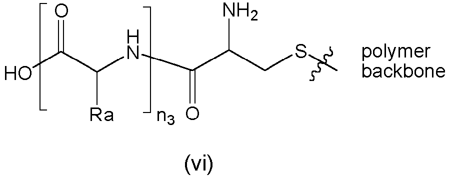 wherein n₃ is an integer from 2 to 19, typically from 3 to 9 or from 3 to 5, and wherein Rₐ is selected at each occurrence from the group consisting of H₂NC(=NH)-NH(CH₂)₃-, H₂N(CH₂)₄-, and (1*H*-imidazol-4-yl)-CH₂-; and the wavy line points out that the oligopeptide is bound to the polymer backbone through -S-.

Where the or each oligopeptide is a compound of formula (vi), the moiety linking the or each oligopeptide to the polymer (e.g. L₁ and/or L₂ and/or L₈, when present, in the first building block) is a bond and the terminal cysteine residue provides a means of coupling the or each oligopeptide to the acrylate terminated compound of the polymer backbone. The thiol functionality provides faster, more efficient and more easily controlled addition to the double bond. By contrast, where the or each oligopeptide is terminated in an amine functionality for coupling, an excess of this compound is required in the coupling step.

Alternatively, the or each oligopeptide may have a net negative charge at pH 7. The or each oligopeptide may comprise naturally occurring amino acids that are negatively charged at pH 7, that is, aspartic acid and glutamic acid. For example, the or each oligopeptide may be selected from polyaspartic acid and polyglutamic acid, each of which may be terminated with cysteine. In this embodiment, the or each oligopeptide may be a compound of formula (vi'): 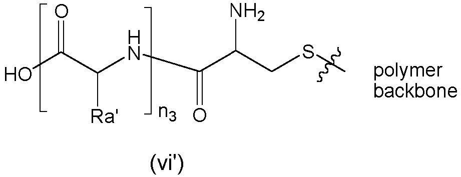 wherein n₃ is an integer from 2 to 19, typically from 3 to 9 or from 3 to 5, and wherein Rₐ' is HO₂C(CH₂)₂- or HO₂C-CH₂-. In this case, the moiety linking the or each oligopeptide to the polymer (e.g. L₁ and/or L₂ and/or L₈, when present, in the first building block) linking the or each oligopeptide to the polymer is a bond as the terminal cysteine residue provides a means of coupling the or each oligopeptide to the acrylate terminated polymer backbone.

Alternatively, the or each oligopeptide may comprise a mixture of naturally occurring amino acids that are negatively charged at pH 7 and naturally occurring amino acids that are positively charged at pH 7.

In one embodiment, optionally in combination with any of the embodiments provided above or below, the polymer building block of formula (I) has the formula (Ic): 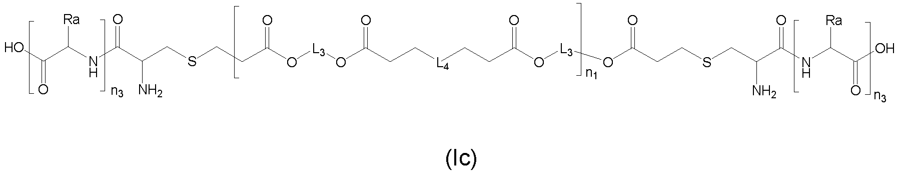 wherein n₃ and Rₐ independently at each occurrence are selected from the lists defined above. In some cases, each occurrence of n₃ is the same and the Rₐ groups are selected such that the sequence of Rₐ groups starting from the sulfur linkage is the same at each end of the compound, that is, n₃ and Rₐ are selected such that the polymer has two-fold symmetry about L₄.

It will be recognized that further methods of attaching an oligopeptide to the polymers defined herein would be available to the skilled person, who would be aware of appropriate nucleophiles for reaction at the terminal acrylate groups.

### Preparation process of the polymers of the invention

The polymer of the invention may be conveniently prepared by using click chemistry. Click chemistry refers to a group of cycloaddition reactions in which two suitable functional groups, referred to as click pairs, react and form a cyclic adduct. The term "click functional group" refers to a functional group which is susceptible of undergoing a click chemistry reaction giving rise to a cyclic adduct when reacted with its corresponding click pair functional group.

Particular examples of click chemistry reactions which can be used for preparing the polymers of the invention include 1,3-dipolar cycloaddition, also known as Huisgen cycloaddition, and the inverse electron demand Diels-Alder reaction (IEDDA).

In the 1,3-dipolar cycloaddition one of the click pairs is a 1,3-dipole, such as an azide moiety, and the other the click pair is a dipolarophile, such as an alkyne moiety. As a result of the cycloaddition a five-membered ring is formed, for example when an azide moiety and an alkyne moiety are used as click pairs, a 1,2,3-triazole is obtained.

A particular 1,3-dipolar cycloaddition is the strain-promoted azide-alkyne 1,3-dipolar cycloaddition (SpAAC), where a strained alkyne moiety is used as a dipolarophile. In this click reaction a stable triazole product is formed by fast and selective cycloaddition of cycloalkyne and azide moiety without the need of reagents, catalysts, or other carefully controlled reaction conditions. Non-limiting examples of alkyne moieties that can be used in the present invention include the ones shown below:

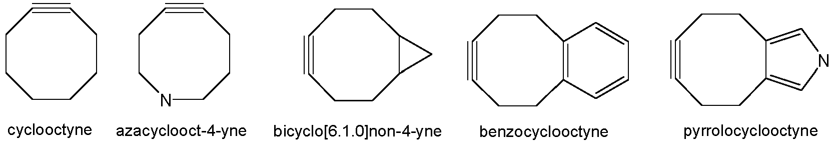

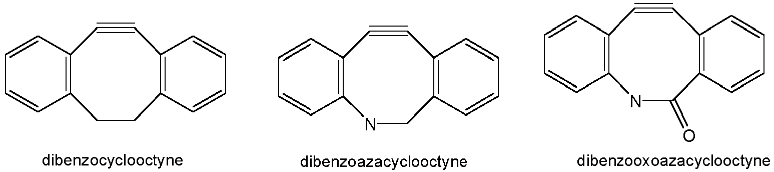

The alkyne moieties may be substituted.

An example of a SpAAC reaction is shown below for illustrative purposes:

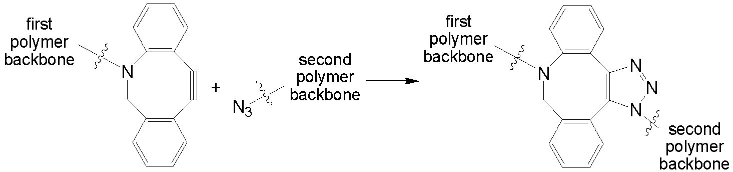

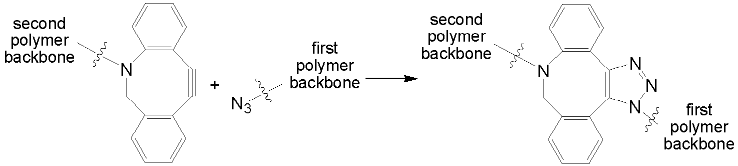

In the inverse electron demand Diels-Alder reaction, the cycloaddition reaction is produced between an electron-rich dienophile such as a strained diene and an electron-poor diene such as a tetrazine moiety as click pairs and a six-membered ring are formed.

Non-limiting examples of strained dienes that may be used in the present invention include the ones shown below:

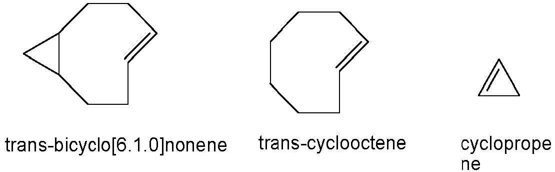

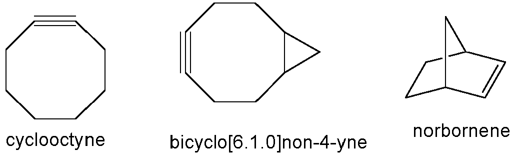

The strained dienes as well as the tetrazine moieties may be substituted.

An example of a IEDDA reaction is shown below for illustrative purposes:

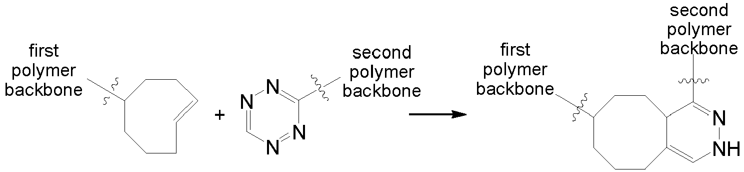

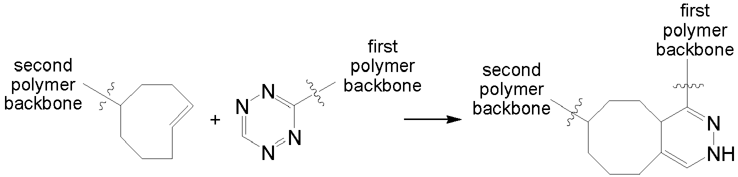

Thus, it also forms part of the invention a process for the preparation of a polymer comprising a first polymer building block and a second polymer building block as defined in the first aspect, wherein the process comprises reacting a first polymer which comprises a lateral chain comprising a first click functional group, and a second polymer which comprises a lateral chain comprising a second click functional group, wherein the first and second click functional groups are click pairs capable of forming a cyclic adduct that binds the first and the second polymers.

In one embodiment, optionally in combination with any of the embodiments provided above or below, the polymer of the first aspect is a polymer wherein the first polymer building block is of formula (I), more particularly of formula (Ia), and the second polymer building block is of formula (II), more particularly of formula (Ila) or formula (IIb).

In another embodiment, optionally in combination with any of the embodiments provided above or below, in the process for the preparation of the polymer of the first aspect, the first polymer comprising a lateral chain comprising a first click functional group is a polymer of formula (I_{A}) 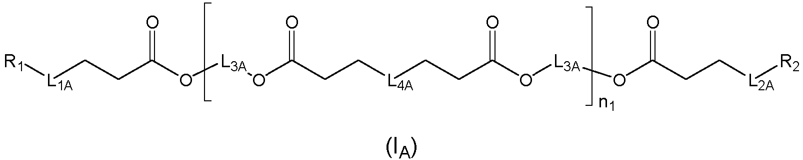 and the second polymer comprising a lateral chain comprising a second click functional group is a polymer of formula (II_{B}) 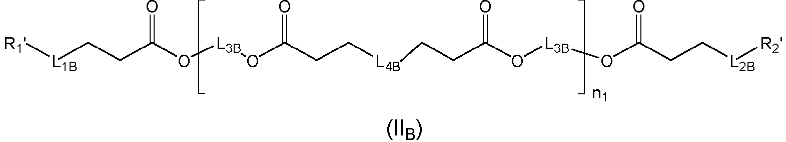 wherein
each L_{3A} is independently selected from the group consisting of alkylene, alkenylene, heteroalkylene, heteroalkenylene, arylene, and heteroarylene; or alternatively
at least one occurrence of L_{3A} is a biradical of formula (i_{1A}): wherein: 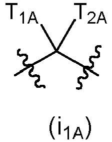
   T_{1A} is a radical of formula (ii_{1A}): 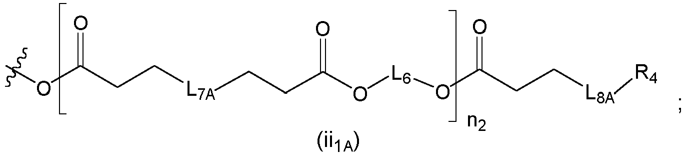
   T_{2A} is selected from the group consisting of H, alkyl and a radical of formula (ii_{1A});
   and the remaining L_{3A} groups are independently selected at each occurrence from the group consisting of alkylene, alkenylene, heteroalkylene, heteroalkenylene, arylene, and heteroarylene;
   each L_{4A} and L_{7A} are independently selected from the group consisting of a biradical of formula (iii_{1A}) and of formula (iv_{1A}): 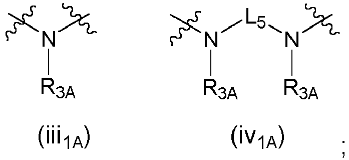
   each L_{1A}, L_{2A}, and L_{8A} are independently selected from the group consisting of O, S, NRₓ, a bond, and a biradical of formula (v_{1A}); 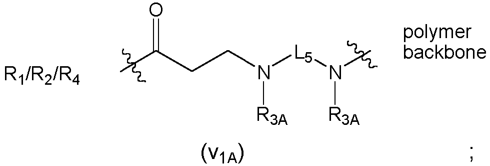
   R₁, R₂ and each R₄ are independently selected from the group consisting of an oligopeptide and R_{y}; wherein at least one of R₁, R₂ and R₄ is an oligopeptide;
   each R_{3A} is independently selected from the group consisting of hydrogen, halogen, alkyl, cycloalkyl, alkenyl, cycloalkenyl, heteroalkyl, heterocycloalkyl, acyl, aryl, heteroaryl, a polyalkylene glycol, -(CH_{z})ₚOH, -(CH₂)₂(OCH₂CH₂)_{q}OH, -(CH₂)ₚNH₂, -(CH₂)₂(OCH₂CH₂)_{q}NH₂, and a click functional group;
   each L_{3B} is independently selected from the group consisting of alkylene, alkenylene, heteroalkylene, heteroalkenylene, arylene, and heteroarylene; or alternatively
   at least one occurrence of L_{3B} is a biradical of formula (i_{1B}): wherein: 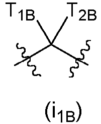
      T_{1B} is a radical of formula (ii_{1B}): 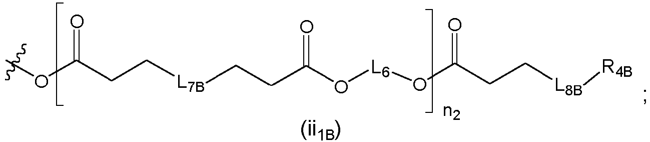
      T_{2B} is selected from the group consisting of H, alkyl and a radical of formula (ii_{1B});
      and the remaining L_{3B} groups are independently selected at each occurrence from the group consisting of alkylene, alkenylene, heteroalkylene, heteroalkenylene, arylene, and heteroarylene;
      each L_{4B} and L_{7B} are independently selected from the group consisting of a biradical of formula (iii_{1B}) and of formula (iv_{1B}): 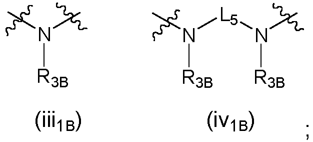
      each L_{1B}, L_{2B}, and L_{3B} are independently selected from the group consisting of O, S, NRₓ, a bond, and a biradical of formula (v_{1B}); 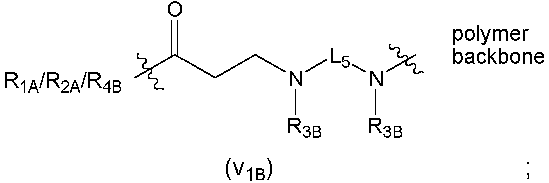
      R_{1B}, R_{2B} and each R_{4B} are independently selected from the group consisting of an oligopeptide, R_{y}, and a moiety selected from the group consisting of an active ingredient moiety, a cell-targeting moiety, a diagnostic agent moiety, and an imaging agent moiety; more particularly the moiety is a cell-targeting moiety;
      each R_{3B} is independently selected from the group consisting of hydrogen, halogen, alkyl, cycloalkyl, alkenyl, cycloalkenyl, heteroalkyl, heterocycloalkyl, acyl, aryl, heteroaryl, a polyalkylene glycol, -(CH_{z})ₚOH, -(CH₂)₂(OCH₂CH₂)_{q}OH, -(CH₂)ₚNH₂, -(CH₂)₂(OCH₂CH₂)_{q}NH₂, a moiety selected from the group consisting of an active ingredient moiety, a cell-targeting moiety, a diagnostic agent moiety, and an imaging agent moiety; more particularly the moiety is a cell-targeting moiety; and a click functional group;
      each L₅ and L₆ are independently selected from the group consisting of alkylene, alkenylene, heteroalkylene, heteroalkenylene, arylene, and heteroarylene;
      each Rₓ and R_{y} is independently selected from the group consisting of hydrogen, halogen, alkyl, cycloalkyl, alkenyl, cycloalkenyl, heteroalkyl, heterocycloalkyl, acyl, aryl, and heteroaryl; and
      n₁ and n₂ are independently an integer from 5 to 1000; p is an integer from 1 to 20; and q, is an integer from 1 to 10;
      provided that:
         - the second polymer (II_{B}) comprises at least an active ingredient, a cell-targeting ligand, a diagnostic agent, an imaging agents, and a combination thereof; more particularly a cell-targeting ligand;
         - at least one of R_{3A}, of the first polymer (I_{A}) is a click functional group, and at least one of R_{3B} of the second polymer (II_{B}) is a click functional group; wherein the click functional groups of the polymer (I_{A}) and the click functional groups of the polymer (II_{B}) are click pairs capable of forming a cyclic adduct binding polymers (I_{A}) and (II_{B}).

More particularly, the process comprises reacting a first polymer of formula (I_{A}) with a second polymer of formula (II_{B}), wherein:
- L_{1A}, L_{2A}, L_{1B}, and L_{2B} are a bond;
- each L_{3A} and L_{3B} are independently selected from the group consisting of alkylene, alkenylene, heteroalkylene, heteroalkenylene, arylene, and heteroarylene; particularly L_{3A} represents alkylene;
- each L_{4A} is a biradical of formula (iii_{1A});
- each L_{4B} is a biradical of formula (iii_{1B});
- each R_{3A} is independently selected from the group consisting of alkyl, -(CH_{z})ₚOH, (CH₂)₂(OCH₂CH₂)_{q}OH, -(CH₂)ₚNH₂, -(CH₂)₂(OCH₂CH₂)_{q}NH₂, and a click functional group;
- each R_{3B} is independently selected from the group consisting of alkyl, -(CH_{z})ₚOH, -(CH₂)₂(OCH₂CH₂)_{q}OH, -(CH₂)ₚNH₂, and -(CH₂)₂(OCH₂CH₂)_{q}NH₂, a moiety selected from the group consisting of an active ingredient moiety, a cell-targeting moiety, a diagnostic agent moiety, an imaging agent moiety; more particularly the moiety is a cell-targeting moiety, and a click functional group;
- R_{1A} and R_{2A} are independently an oligopeptide;
- R_{1B} and R_{2B} are independently an oligopeptide or a moiety selected from the group consisting of an active ingredient moiety, a cell-targeting moiety, a diagnostic agent moiety, an imaging agent moiety; more particularly the moiety is a cell-targeting moiety;
- p is an integer from 1 to 20, and q is an integer from 1 to 10.
provided that:
- the second polymer (II_{B}) comprises at least an active ingredient, a cell-targeting ligand, a diagnostic agent, an imaging agents, and a combination thereof; more particularly a cell-targeting ligand and/or an oligopeptide;
- at least one of R_{3A} of the first polymer (I_{A}) is a click functional group, and at least one of R_{3B} of the second polymer (II_{B}) is a click functional group; wherein the click functional groups of the polymer (I_{A}) and the click functional groups of the polymer (II_{B}) are click pairs capable of forming a cyclic adduct binding polymers (I_{A}) and (II_{B}).

All the embodiments provided above for the first polymer building block of formula (I) apply in an analogous manner to its corresponding precursor of formula (I_{A}) and all the embodiments provided above for the second polymer building block of formula (II) apply in an analogous manner to its corresponding precursor of formula (II_{A}).

In another embodiment, optionally in combination with any of the embodiments provided above or below, the molecular weight of the first polymer of formula (I_{A}) is from 500 to 150000 g/mol, more particularly from 700 to 100000 g/mol, even more particularly from 2000 to 50000 g/mol, and even more particularly from 5000 to 40000 g/mol.

In another embodiment, optionally in combination with any of the embodiments provided above or below, the molecular weight of the second polymer of formula (II_{B}) is from 500 to 150000 g/mol, more particularly from 700 to 100000 g/mol, even more particularly from 2000 to 50000 g/mol, and even more particularly from 5000 to 40000 g/mol.

In another embodiment, optionally in combination with any of the embodiments provided above or below, in the process for the preparation of the polymer of the first aspect, the first and the second click functional groups are selected from the group consisting of an alkyne moiety, an azide moiety, a diene moiety, and a tetrazine moiety.

In a more particular embodiment, the first click functional group is an alkyne moiety, and the second click functional group is an azide moiety. In an alternative more particular embodiment, the first click functional group is an azide moiety, and the second click functional group is an alkyne moiety.

In another more particular embodiment, the first click functional group is an alkyne or diene moiety, and the second click functional group is a tetrazine moiety. In an alternative more particular embodiment, the first click functional group is a tetrazine moiety, and the second click functional group is an an alkyne or diene moiety.

The first polymer of formula (I_{A}) may be prepared from a polymer of formula (IV_{A}) by reaction of the terminal acrylate groups with R₁L_{1A}H and R₂L_{2A}H:

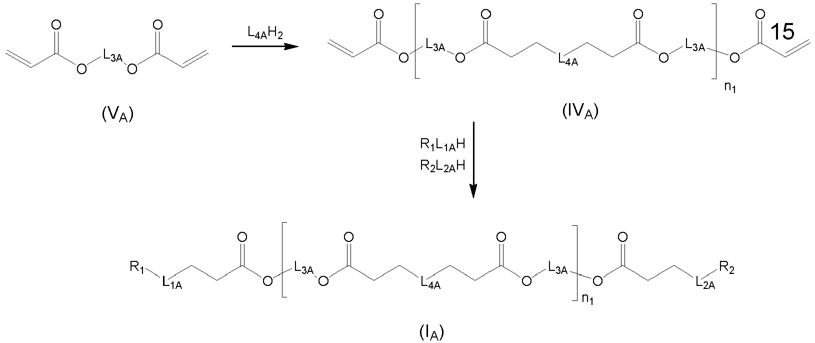

Each L_{1A}, L_{2A}, and L_{8A} is selected to facilitate coupling of the end-modifying groups R₁ and R₂ to the PBAE polymer. Each L_{1A}, L_{2A} and L_{8A} may be a bond, for example where the end-modifying group is an oligopeptide that comprises a terminal cysteine residue.

Further, the polymer of formula (IV_{A}) may be prepared by reacting an acrylate of formula (V_{A}) with one or more amines of formula L_{4A}H₂ under suitable conditions well known in the art.

The second polymer of formula (II_{B}) may be prepared in an analogous manner from the corresponding acrylates and amines.

It also forms part of the present invention the invention a polymer comprising a first polymer building block and a second polymer building block as defined in the first aspect which is obtainable by a process comprising reacting a first polymer which comprises a lateral chain comprising a first click functional group, and a second polymer which comprises a lateral chain comprising a second click functional group, wherein the first and second click functional groups are click pairs capable of forming a cyclic adduct that binds the first and the second polymers.

All the embodiments provided above for the polymers of the first aspect of the invention, are also embodiments of the polymers defined by their preparation process.

The expression "obtainable by" is used herein for defining the polymer comprising two building blocks by its preparation process and refers to the polymer that can be obtained through the preparation process disclosed herein. For the purposes of the invention, the expressions "obtainable", "obtained" and similar equivalent expressions are used interchangeably and, in any case, the expression "obtainable" encompasses the expression "obtained".

### Nanoparticles

The present invention further provides nanoparticles comprising the polymer comprising the first and the second polymer building blocks as defined herein. All the embodiments provided above for the polymers of the first aspect of the invention, are also embodiments of the nanoparticles of the second aspect of the invention.

Such nanoparticles are suitable for encapsulation of a particular molecule (compound of interest), such as a diagnostic and imaging reagents or an active agent or a cell-targeting ligand, and show improved pharmacological properties.

Diagnostic and imaging agents include contrast agents, magnetic materials, agents sensitive to light, radiolabels, and fluorescent compounds, such as carboxyfluorescein. Such agents may be used for biodistribution studies *in vitro* and *in vivo.* Delivery of nanoparticles of the present invention to the brain has been demonstrated by such studies. For example, paclitaxel-loaded nanoparticles comprising surface-modifying agents have been detected in the brain in biodistribution studies *in vivo.* Moreover, fluorescently labelled nanoparticles can be used in a cellular study simulating the blood-brain barrier.

The active agent(s) may be present within the nanoparticles or on the surfaces of the nanoparticles. Typically, the active agent is present within the nanoparticles. The interaction between the active agent(s) and the nanoparticle is typically non-covalent, for example, hydrogen bonding, electrostatic interaction or physical encapsulation. Typically, the interaction is electrostatic.

The nanoparticles are biocompatible and sufficiently resistant to their environment of use that a sufficient amount of the nanoparticles remain substantially intact after entry into the mammalian body so as to be able to reach the desired target and achieve the desired physiological effect. The polymers described herein are biocompatible and particularly biodegradable.

Herein, the term 'biocompatible' describes as substance which may be inserted or injected into a living subject without causing an adverse response. For example, it does not cause inflammation or acute rejection by the immune system that cannot be adequately controlled. It will be recognized that "biocompatible" is a relative term, and some degree of immune response is to be expected even for substances that are highly compatible with living tissue. An in vitro test to assess the biocompatibility of a substance is to expose it to cells; biocompatible substances will typically not result in significant cell death (for example, >20%) at moderate concentrations (for example, 29 µg/10<4 >cells).

Herein, the term 'biodegradable' describes a polymer which degrades in a physiological environment to form monomers and/or other non-polymeric moieties that can be reused by cells or disposed of without significant toxic effect. Degradation may be biological, for example, by enzymatic activity or cellular machinery, or may be chemical, typically a chemical process that takes place under physiological conditions. Degradation of a polymer may occur at varying rates, with a half-life in the order of days, weeks, months, or years, depending on the polymer or copolymer used. The components particularly do not induce inflammation or other adverse effects in vivo. In certain particular embodiments, the chemical reactions relied upon to break down the biodegradable compounds are uncatalysed.

Herein, the term "nanoparticles" refers to a solid particle with a diameter of from about 1 to about 1000 nm, particularly from 50 to 800nm, particularly from 100 to 500 nm. The mean diameter of the nanoparticles of the present invention may be determined by methods known in the art, particularly by light scattering. In particular, the invention relates to nanoparticles that are solid particles with a diameter of from about 1 to about 1000nm when analysed by dynamic light scattering at a scattering angle of 173° and at a temperature of 25 °C, using a sample appropriately diluted with filtered water and a suitable instrument such as the Zetasizer^{™} instruments from Malvern Instruments (UK). Where a particle is said to have a diameter of x nm, there will generally be a distribution of particles about this mean, but at least 50% by number (e.g. >60%, >70%, >80%, >90%, or more) of the particles will have a diameter within the range x±20%.

Nanoparticles of the present disclosure may be formed with high active agent content and high encapsulation efficiency. Herein, the active agent encapsulation efficiency refers to the active agent incorporated into the nanoparticles as a weight percentage of the total active agent used in the method of preparation of the active agent-containing nanoparticles. It is typically up to and including 95%, more typically from 70% to 95%.

Herein, active agent entrapment refers to the weight percentage of the active agent in the active agent-loaded nanoparticles. Active agent entrapment is particularly at least 2 wt %, more particularly at least 5 wt %, more particularly at least 10 wt % and typically in the range of from 2 wt % to 20 wt %, more particularly from 5 wt % to 20 wt %, more particularly from 10 wt % to 20 wt %.

In one embodiment, optionally in combination with any of the embodiments provided above or below, the compound of interest is an active ingredient.

In one embodiment, optionally in combination with any of the embodiments provided above or below, the compound of interest, particularly the active ingredient, is negatively charged at biological pH.

In an alternative embodiment, optionally in combination with any of the embodiments provided above or below, the compound of interest, particularly the active ingredient, is positively charged at biological pH.

The "positively charged at biological pH" in the context of the invention is meant to include any compound, particularly any pharmacologically active substance, that bear negative charges in the molecule in an aqueous solution at a biological pH (about 7).

The "negatively charged at biological pH" in the context of the invention is meant to include any compound, particularly any pharmacologically active substance, that bear negative charges in the molecule in an aqueous solution at a biological pH (about 7). In one embodiment, the anionic nature can be imparted from one or more functional groups selected from the group consisting of carboxyl, phosphate and sulphate groups.

In one embodiment of the present invention, optionally in combination with any of the embodiments provided above or below, the negatively charged compound of interest is a polyanionic small molecule, a nucleic acid, polypeptide or protein. As used herein, the term "small molecule" refers to organic compounds, whether naturally-occurring or artificially created (e.g., via chemical synthesis) that have relatively low molecular weight and that are not proteins, polypeptides, or polynucleotides. Typically, small molecules have a molecular weight of less than about 1500 g/mol. Examples of the polyanionic small molecules may include heparin, calcitonin, etc.

The nucleic acid may be a nucleic acid drug, such as deoxyribonucleic acid, ribonucleic acid, or a polynucleotide derivative wherein the backbone, sugar or base is chemically modified or the end of the nucleic acid is modified. Particularly, it may be one or more nucleic acid selected from the group consisting of RNA, DNA (such as a gene or oligonucleotide), siRNA (small interfering RNA), an aptamer, antisense oligodeoxynucleotide (ODN), antisense RNA, ribozyme, DNAzyme, ASO, ribozyme, oligonucleotide RNA inhibitor, immune modulating oligonucleotide or other desired negatively charged nucleic acid.

Also, in order to increase the stability of the nucleic acid in blood or weaken the immune response, the backbone, sugar or base of the nucleic acid may be chemically modified or the end of the nucleic acid may be modified. Specifically, a portion of the phosphodiester bond of the nucleic acid may be substituted by a phosphorothioate or boranophosphate bond, or the nucleic acid may include at least one nucleotide wherein various functional groups such as a methyl group, a methoxyethyl = 30 group or fluorine are introduced in the 2'-OH position of some riboses. Therefore, the polynucleotide may be derived from natural nucleosides (i.e., adenosine, thymidine, guanosine, cytidine, uridine, inosine, xanthosine, deoxyadenosine, deoxythymidine, deoxyguanosine, deoxyinosine, and deoxycytidine), nucleoside analogues (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), or mixtures thereof. The nucleotides may be derived from chemically modified bases, biologically modified bases (e.g., methylated bases), intercalated bases, unmodified or modified sugars (e.g., 2'-fluororibose, ribose, 2'-deoxyribose, arabinose, and hexose), and/or unmodified or modified phosphate groups (e.g., phosphorothioates and 5'-N-phosphoramidite linkages).

Typically, a polynucleotide comprises at least three nucleotides. Particularly, the polynucleotide is 20-30 nucleotides in length, more particularly 20-25 nucleotides in length, for example, 22 nucleotides in length.

In another embodiment, optionally in combination with any of the embodiments provided above or below, one or more ends of the nucleic acid may be modified with one or more selected from the group consisting of cholesterol, tocopherol, a fatty acid having 10-24 carbon atoms, and any analogue, derivative and metabolite thereof. For example, siRNA may be modified at the 5' end or at the 3' end or at both ends of the sense and/or antisense strand, and particularly at the end of the sense strand.

In another embodiment, optionally in combination with any of the embodiments provided above or below, the nanoparticle comprises a polymeric shell comprising the polymer(s) which comprise the two building blocks; and a core comprising the compound(s).

In another embodiment, optionally in combination with any of the embodiments provided above or below, the nanoparticle comprises a polymer wherein the first building block comprises one or more oligopeptides positively charged at pH 7, and the second building block comprises one or more oligopeptides negatively charged at pH 7. These nanoparticles are especially advantageous because allow to fine-tune the targeting to the desired cell.

In another embodiment, optionally in combination with any of the embodiments provided above or below, the nanoparticle comprises a polymer as defined in the first aspect, wherein the first polymer building block is of formula (I), more particularly of formula (Ia), and the second polymer building block is of formula (II), more particularly of formula (Ila) or formula (IIb).

In another embodiment, optionally in combination with any of the embodiments provided above or below, the nanoparticle further comprises one or more polymers of formula (III) or a pharmaceutically acceptable salt thereof: 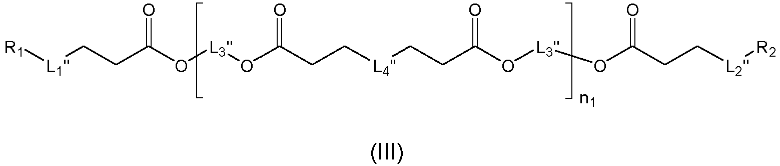 wherein:
each L₃" is independently selected from the group consisting of alkylene, alkenylene, heteroalkylene, heteroalkenylene, arylene, and heteroarylene; or alternatively
at least one occurrence of L₃" is a biradical of formula (i₁"): 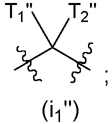 wherein:
   T₁" is a radical of formula (ii₁"): 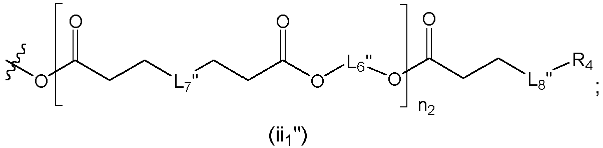
   T₂" is selected from the group consisting of H, alkyl and a radical of formula (ii₁");
and the remaining L₃ groups are independently selected at each occurrence from the group consisting of alkylene, alkenylene, heteroalkylene, heteroalkenylene, arylene, and heteroarylene;
each L₄" and L₇" are independently selected from the group consisting of a biradical of formula (iii₁") and of formula (iv₁"): 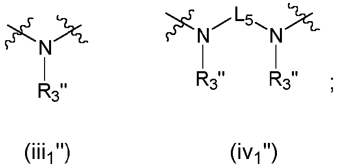
each L₁", L₂", and L₈" are independently selected from the group consisting of O, S, NRₓ, a bond, and a biradical of formula (v₁"); 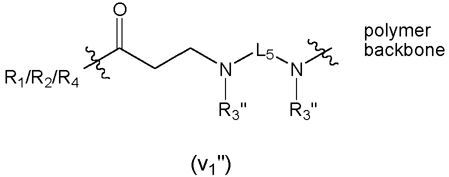
each L₅ and L₆ are independently selected from the group consisting of alkylene, alkenylene, heteroalkylene, heteroalkenylene, arylene, and heteroarylene;
R₁, R₂ and each R₄ are independently selected from the group consisting of an oligopeptide and R_{y};
each R₃" is independently selected from the group consisting of hydrogen, halogen, alkyl, cycloalkyl, alkenyl, cycloalkenyl, heteroalkyl, heterocycloalkyl, acyl, aryl, heteroaryl, a polyalkylene glycol, -(CH_{z})ₚOH, -(CH₂)₂(OCH₂CH₂)_{q}OH, -(CH₂)ₚNH₂, and -(CH₂)₂(OCH₂CH₂)_{q}NH₂;
each Rₓ and R_{y} is independently selected from the group consisting of hydrogen, halogen, alkyl, cycloalkyl, alkenyl, cycloalkenyl, heteroalkyl, heterocycloalkyl, acyl, aryl, and heteroaryl; and
n₁ and n₂ are independently an integer from 5 to 1000; p is an integer from 1 to 20; and q, is an integer from 1 to 10.

In another embodiment, optionally in combination with any of the embodiments provided above or below, the nanoparticle further comprises one or more polymers of formula (IV) or a pharmaceutically acceptable salt thereof: 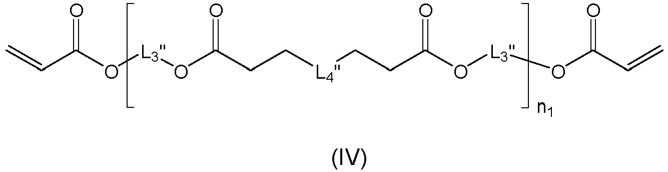 wherein L₃", L₄" and n₁ are as defined herein.

In one particular embodiment, optionally in combination with any of the embodiments provided above or below, in the polymer of formula (III) or formula (IV), each L₃" is independently selected from the group consisting of alkylene, alkenylene, heteroalkylene, heteroalkenylene, arylene, and heteroarylene; particularly L₃" represents alkylene.

In another particular embodiment, optionally in combination with any of the embodiments provided above or below, in the polymer of formula (III) or formula (IV), each L₄" is a biradical of formula (iii₁"). More particularly, the polymer of formula (III) or formula (IV) comprises at least two different L₄ biradicals of formula (iii₁").

In another particular embodiment, optionally in combination with any of the embodiments provided above or below, in the polymer of formula (III) or formula (IV), each R₃" is independently selected from the group consisting of alkyl, -(CH_{z})ₚOH, -(CH₂)₂(OCH₂CH₂)_{q}OH, -(CH₂)ₚNH₂, and -(CH₂)₂(OCH₂CH₂)_{q}NH₂, wherein p is an integer from 1 to 20, and q is an integer from 1 to 10, more particularly p and q are independently an integer from 1 to 10.

In another particular embodiment, optionally in combination with any of the embodiments provided above or below, in the polymer of formula (III) or formula (IV), at least one R₃" is alkyl and at least one R₃" is -(CH_{z})ₚOH, wherein p is an integer from 1 to 10.

In another particular embodiment, optionally in combination with any of the embodiments provided above or below, in the polymer of formula (III), L₁" and L₂" are a bond.

In the polymer of formula (III) at least one of R₁, R₂ and R₄ is an oligopeptide. In one embodiment, optionally in combination with any of the embodiments provided above or below, in the polymer of formula (III), one of R₁, R₂ and R₄ is an oligopeptide and the remaining radicals of R₁, R₂ and R₄ are independently R_{y}. In another embodiment, optionally in combination with any of the embodiments provided above or below, in the polymer of formula (III), two of R₁, R₂ and R₄ are independently an oligopeptide and the remaining radical of R₁, R₂ and R₄ is R_{y}. In another embodiment, optionally in combination with any of the embodiments provided above or below, in the polymer of formula (III), R₁, R₂ and R₄ are independently an oligopeptide.

In another particular embodiment, optionally in combination with any of the embodiments provided above or below, in the polymer of formula (III), R_{y} is selected from the group consisting of hydrogen, -(CH₂)ₘNH₂, -(CH₂)ₘNHMe, -(CH₂)ₘOH, -(CH₂)ₘCH₃, -(CH₂)₂(OCH₂CH₂)ₘNH₂, -(CH₂)₂(OCH₂CH₂)ₘOH, and -(CH₂)₂(OCH₂CH₂)ₘCH₃ wherein m is an integer from 1 to 20, for example from 1 to 5. Particularly, R_{y} is selected from the group consisting of -(CH₂)ₘNH₂, -(CH₂)ₘNHMe, and-(CH₂)₂(OCH₂CH₂)ₘNH₂.

In another particular embodiment, optionally in combination with any of the embodiments provided above or below, in the polymer of formula (III), R₁ and R₂ are independently an oligopeptide, more particularly R₁ and R₂ are the same oligopeptide, more particularly, R₁ and R₂ are the same positively charged oligopeptide at pH 7, even more particularly R₁ and R₂ are an oligopeptide of formula of formula (vi): 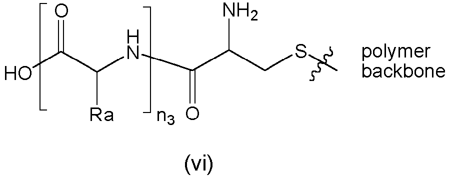 wherein n₃ is an integer from 2 to 19, and each Rₐ is selected from the group consisting of H₂NC(=NH)-NH(CH₂)₃-, H₂N(CH₂)₄-, and (1*H*-imidazol-4-yl)-CH₂-.

In another particular embodiment, optionally in combination with any of the embodiments provided above or below, the polymer of formula (III) is a polymer of formula (IIIa), wherein:
- each L₃" is independently selected from the group consisting of alkylene, alkenylene, heteroalkylene, heteroalkenylene, arylene, and heteroarylene; particularly L₃" represents alkylene;
- each L₄" is a biradical of formula (iii₁");
- each R₃" is independently selected from the group consisting of alkyl, -(CH_{z})ₚOH, -(CH₂)₂(OCH₂CH₂)_{q}OH, -(CH₂)ₚNH₂, and -(CH₂)₂(OCH₂CH₂)_{q}NH₂, wherein p is an integer from 1 to 20, and q is an integer from 1 to 10; more particularly at least one R₃" is alkyl and at least one R₃" is -(CH_{z})ₚOH, wherein p is an integer from 1 to 10;
- L₁" and L₂" are a bond;
- R₁ and R₂ are independently an oligopeptide, more particularly R₁ and R₂ are the same oligopeptide, more particularly, R₁ and R₂ are the same positively charged oligopeptide at pH 7, even more particularly R₁ and R₂ are an oligopeptide of formula of formula (vi).

In another embodiment, optionally in combination with any of the embodiments provided above or below, the polymer of formula (Illa) comprises at least two different L₄" biradicals of formula (iii₁"), more particularly wherein at least one R₃" is alkyl and at least one R₃" is -(CH₂)ₚOH.

In another particular embodiment, optionally in combination with any of the embodiments provided above or below, the polymer of formula (IV) is a polymer of formula (IVa), wherein:
- each L₃" is independently selected from the group consisting of alkylene, alkenylene, heteroalkylene, heteroalkenylene, arylene, and heteroarylene; particularly L₃" represents alkylene;
- each L₄" is a biradical of formula (iii₁");
- each R₃" is independently selected from the group consisting of alkyl, -(CH₂)ₚOH, -(CH₂)₂(OCH₂CH₂)_{q}OH, -(CH₂)ₚNH₂, and -(CH₂)₂(OCH₂CH₂)_{q}NH₂, wherein p is an integer from 1 to 20, and q is an integer from 1 to 10; more particularly at least one R₃" is alkyl and at least one R₃" is -(CH₂)ₚOH, wherein p is an integer from 1 to 10;

In another embodiment, optionally in combination with any of the embodiments provided above or below, the polymer of formula (IVa) comprises at least two different L₄" biradicals of formula (iii₁"), more particularly wherein at least one R₃" is alkyl and at least one R₃" is -(CH₂)ₚOH.

In another embodiment, optionally in combination with any of the embodiments provided above or below, the nanoparticle comprises:
i) a polymeric shell comprising the polymer as defined in the first aspect and one or more other polymers, particularly selected from a polymer of formula (III), a polymer of formula (IV) and a combination thereof, and
ii) a core comprising one or more compounds.

In another embodiment, optionally in combination with any of the embodiments provided above or below, the nanoparticle comprises:
a) a polymer as defined in the first aspect, wherein the first polymer building block is of formula (I), more particularly of formula (Ia), and the second polymer building block is of formula (II), more particularly of formula (Ila) or formula (IIb), and
b) a polymer of formula (III).

In another embodiment, optionally in combination with any of the embodiments provided above or below, the nanoparticle comprises:
a) a polymer as defined in the first aspect, wherein the first polymer building block is of formula (I), more particularly of formula (Ia), and the second polymer building block is of formula (II), more particularly of formula (Ila) or formula (IIb), and
b) a polymer of formula (IV).

In another embodiment, optionally in combination with any of the embodiments provided above or below, the nanoparticle comprises:
a) a polymer as defined in the first aspect, wherein the first polymer building block is of formula (I), more particularly of formula (Ia), and the second polymer building block is of formula (II), more particularly of formula (Ila) or formula (Ilb),
b) a polymer of formula (III), and
c) a polymer of formula (IV).

The present invention further provides methods of encapsulating a compound of interest, such as an active ingredient, in a polymeric matrix.

Thus, the invention provides a method of encapsulating a compound of interest which comprises the steps of:
i) providing a compound of interest;
ii) providing a polymer as defined in the first aspect wherein the first polymer building block is of formula (I), more particularly of formula (Ia), and the second polymer building block is of formula (II), more particularly of formula (IIa) or formula (IIb), and optionally one or more other polymers, particularly selected from a polymer of formula (III), a polymer of formula (IV) and a combination thereof; and
iii) contacting the agent and the polymer under suitable conditions to form nanoparticles.

The present invention also provides an alternative method of encapsulating a compound of interest, which comprises the steps of:
i) providing a compound of interest, particularly negatively charged;
ii) providing a first polymer which comprises a lateral chain comprising a first click functional group, particularly a polymer of formula (I_{A}), and optionally one or more other polymers, particularly selected from a polymer of formula (III), a polymer of formula (IV) and a combination thereof;
iii) contacting the agent and the polymer under suitable conditions to form nanoparticles; and
iv) contacting the nanoparticles with a second polymer which comprises a lateral chain comprising a second click functional group, particularly a polymer of formula (II_{B}), wherein the first and second click functional groups are click pairs capable of forming a cyclic adduct that binds the first and the second polymers.

The skilled person, using their general knowledge, can routinely adjust the parameters to obtain the nanoparticles. For example, the step (iii) above may comprise a) spray drying a mixture of the agent and the polymers, b) double emulsion solvent evaporation techniques or c) a phase inversion technique. An illustrative example is provided below, in the Examples.

### Pharmaceutical Compositions

The compositions of the invention comprise a therapeutically effective amount of the polymers as defined in the first aspect or the nanoparticles as defined in the second aspect, when they incorporate an active ingredient, together with pharmaceutically acceptable excipients.

The expression "therapeutically effective amount" as used herein, refers to the amount of the polymers or nanoparticles of the invention that, when administered passes to blood stream and is sufficient to prevent development of, or alleviate to some extent, one or more of the symptoms of the disease which is addressed. The particular dose will of course be determined by the particular circumstances surrounding the case, including the compound administered, the route of administration, the particular condition being treated, and the similar considerations.

The term "cosmetically effective amount" as used herein, refers to the effective amount the polymers or nanoparticles of the invention that, when administered, is intended for aesthetic purposes e.g. to improve the appearance or to beautify, preserve, condition, cleanse, color or protect the skin, nails or hair without a medical application.

The term "pharmaceutically acceptable" refers to that excipients suitable for use in the pharmaceutical technology for preparing compositions with medical use. The term "cosmetically acceptable" refers to excipients which are appropriate for use in human skin contact without toxicity, incompatibility, instability, inappropriate allergic response, among others.

The expression "excipients " refers to acceptable materials, compositions or vehicles. Each component must be pharmaceutically acceptable in the sense of being compatible with the other ingredients of the composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The formulations of the pharmaceutical compositions described herein may be prepared by any method known or hereafter developed in the art of pharmacology. In general, such preparatory methods include the step of bringing the polymer or nanoparticles of the invention into association with an excipient and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the polymer or the nanoparticles of the invention.

The relative amounts of the active ingredient (i.e., the peptide as defined in any of the previous aspects and embodiments), the acceptable excipients, and/or any additional ingredients in the composition of the invention will vary, depending upon the identity, size, and/or condition of the subject treated and further depending upon the route by which the composition is to be administered.

Acceptable excipients used in the manufacture of these compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in the inventive formulations. Excipients such as cocoa butter and suppository waxes, coloring agents, coating agents, sweetening, flavoring, and perfuming agents can be present in the composition, according to the judgment of the formulator.

Particular pharmaceutically acceptable vehicles include water and physiologically acceptable aqueous solutions containing salts and/or buffers, for example, saline or phosphate- buffered saline. Optionally, the pharmaceutically acceptable vehicle is a biological fluid. A liquid vehicle may be removed by, for example, lyophilization, evaporation or centrifugation for storage or to provide a powder for pulmonary or nasal administration, a powder for suspension for infusion, or tablets or capsules for oral administration. Administration of the compositions described herein can be via any of the accepted modes of administration for such compositions including, but not limited to, orally, sublingually, subcutaneously, intravenously, intratumorally, intranasally, topically, transdermally, intraperitoneally, intramuscularly, intrapulmonarilly, vaginally, rectally, or intraocularly. In an embodiment, the composition is for oral or parenteral. In another embodiment, the composition is administered intravenously or intratumorally.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### Materials

Reagents and solvents used for polymer synthesis were purchased from Sigma Aldrich and Panreac. All chemicals were reagent grade. Deuterated solvents were purchased from Sigma Aldrich.

Oligopeptides used for polymer modification (H-Cys-Lys-Lys-Lys-NH2, H-His-His-His-Cys-NH2) were obtained from Ontores (Shangai) with a purity higher than 97%. Antibodies for flow cytometry were purchased from eBioscience^{™} Fixable Viability Dye eFluor^{™} 780, catalog number: 65-0865-18; PE Anti-Hu CD14, clone 61D3, catalog number: 12-0149-42; PE-Cyanine7 Anti-Hu HLA-DR, clone L243, catalog number: 25-9952-42, and PE-Cyanine5 Anti-Hu CD86, clone IT2.2, catalog number: 15-0869-42. mRNA was purchased from Cellerna Bioscience Pure Boost^{™} eGFP (2001M-1MG) and PureBoost^{™} OVA mRNA (6001U-1MG).

Plasmid pMaxGFP (3486 pb) was obtained in the group and amplified in DH5α *E.coli* strain using QIAGEN Plasmid Giga kit (ref 12191) from Qiagen.

### Cell lines

JAWSII (ATCC CRL-11904) cells were cultured in Eagle's minimum essential medium supplemented with 20% (v/v) heat-inactivated fetal bovine serum (FBS); 1 mmol L-1 sodium pyruvate, 4 mmol L-1 glutamine, and 5 ng mL-1 of granulocyte-macrophage colony stimulatory factor (GM-CSF) and 100 U mL-1 of penicillin-streptomycin.

### Structural characterization

¹H and ¹³C NMR spectra were recorded in a Varian 500 MHz spectrometer operated at 298 K. For ¹H NMR, the central peak of the DMSO-d₆ multiplet (2.49 ppm) was used as a reference. For ¹³C NMR, the central peak of the DMSO-d₆ multiplet (39.5 ppm) was used as a reference. All data was processed and analyzed using the MestreNova 8.0.0 software.

UHPLC-QTOF samples were diluted 1/1000 in H₂O and filtered with PVDF 0.22 µm filters. UHPLC-QTOF was performed using a UHPLC ExionLC^{™} AD System (AB Sciex, USA) coupled with an X500B QTOF System (MS/MS) (AB Sciex, USA) with electrospray ionization (ESI) in positive mode. Chromatographic separation was done using a bioZen ^{™} 1.6 µm Peptide PS-C18 column, 150 x 2.1 mm, from Phenomenex. Mobile phase A was 0.1% trifluoroacetic acid in Milli-Q water and B was acetonitrile. A linear gradient program at a flow rate of 0.4 mL/min was used as follows: 0-10 min, from 5 to 35% (B); 10-11 min, from 35 to 100% (B); then 1.5 min to 100% (B) and back to 5% (B) for 2 min. The injection volume was set at 50 µL.

ESI source parameters: spray voltage, +5500 V; source temperature, 500 _{°}C; nebulizer and auxiliary gas flow (N₂), 60 psi and 60 psi; curtain gas flow (N₂), 35 psi. Mass spectrometry analyses were performed applying a data-independent scan experiment (SWATH^{®}) which uses a combination of a full-scan TOF spectrum (declustering potential (DP), 80 V; collision energy (CE), 10 V) with TOF MS/MS fragmentation using fixed Q1 transmission windows. Data were processed using SCIEX OS 2.2 acquisition software.

### Biophysical characterization of nanoparticles (NPs)

Hydrodynamic diameter (nm), polydispersity index (PDI), and surface charge of nanoparticles were measured at 25 °C, 633 nm laser wavelength and 173° signal detector, using a ZetaSizer Nano ZS (Malvern Instruments Ltd, Malvern, UK). For size measurements, nanoparticles prepared at 0.25 mg/mL of mRNA were used and for surface charge, 1/10 dilution. Three measurements of each nanoparticle batch were performed with 20 runs per measurement, considering the intensity approximation. Results correspond to the mean ± standard deviation of at least three independent nanoparticle batches. The hydrodynamic size was also measured by nanoparticle tracking analysis (NTA), using a NanoSight NS300 from Malvern, equipped with a fluorescent laser (λ = 488 nm). Results correspond to the mean ± standard deviation of at least three independent nanoparticle batches. Encapsulation efficiency was measured by the Quanti-It RiboGreen RNA assay kit (ThermoFisher Scientific) following manufacturer instructions and agarose gel retardation assay.

### 1. Synthesis of Click Chemistry Building Blocks

### 1.1. C6-DBCO-CK3 (33)

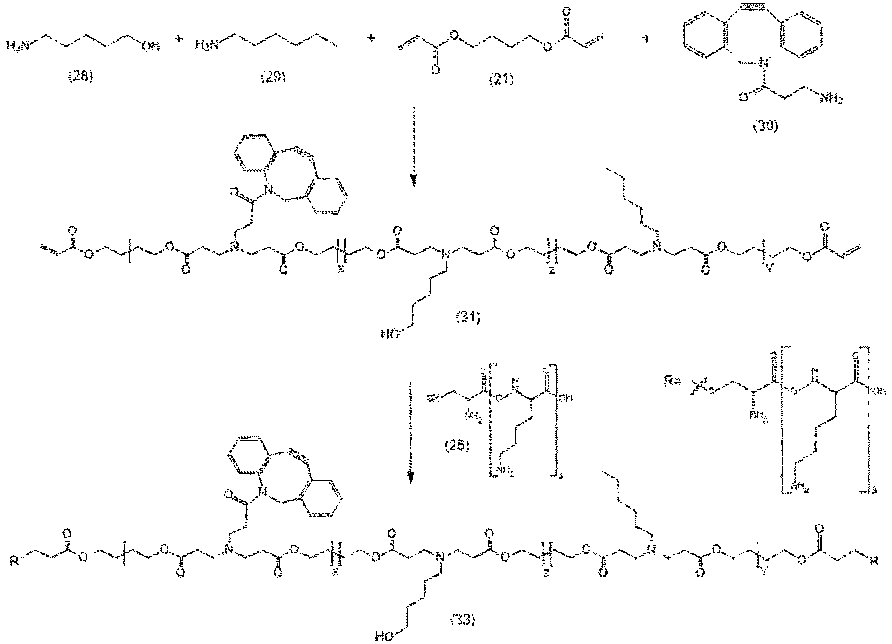

56.7 mg of 1,4-butanediol diacrylate (21), 15.8 mg of hexylamine (29) (0.5 eq), 12.2 mg of 5-aminopentanol (28) (0.3 eq) and 13.3 mg of DBCO-NH₂ (30) (0.2 eq) were mixed and stirred for 96 h at 70 °C. The resulting reaction mixture was purified by dropwise into acetone: diethyl ether (1:1) and several washes were performed. Finally, the precipitated polymer was dried over the vacuum or freeze-dried. The final product (31) was kept at -20 °C. ¹H-NMR (400MHz, d₆-DMSO): δ = 7.32 (m, DBCO-ring), 5.91 (d, CH₂=CH-), 5.03-5.00 (m, (DBCO) CH-CH=CH-), 3.98 (t, -CH₂-CH₂-O-), 3.56 (t, CH₂-CH₂-OH), 2.78 (m, N-CH₂-CH₂-(C=O)-N-), 2.62 (m, -CH₂-CH₂-N-), 2.33 (m, -N-CH₂-CH₂-C(=O)- O), 1.59 (m, -O-CH₂-CH₂-CH₂-CH₂-O), 1.19 (m, - CH₂-CH₂-CH₂-CH₂-OH, N-(CH₂)₂-CH₂-(CH₂)₂-OH), 0.82 (t, CH₂-CH₂-CH₃). It was determined that the structure comprised based on 2 side chains of DBCO-NH2 (X), 5-6 side chains of total hexylamine and aminopentanol (Y and Z), and 8-9 chains of 1,4-butanediol diacrylate. The molecular weight was about 2850 g/mol.

Oligo peptide Cys-Lys-Lys-Lys (CK3) (25) was added to polymer (31) at 37 °C for 24 h. The generated oligo-capped product (33) was resuspended at 100 mg/mL in DMSO until the next use and kept at -20 °C. The chemical structure of product (33) was confirmed by the absence of the terminated-acrylate signals between 5.8- 6.3 ppm in the ¹H-NMR spectra.

### 1.2. Man2-N3-Cap (37)

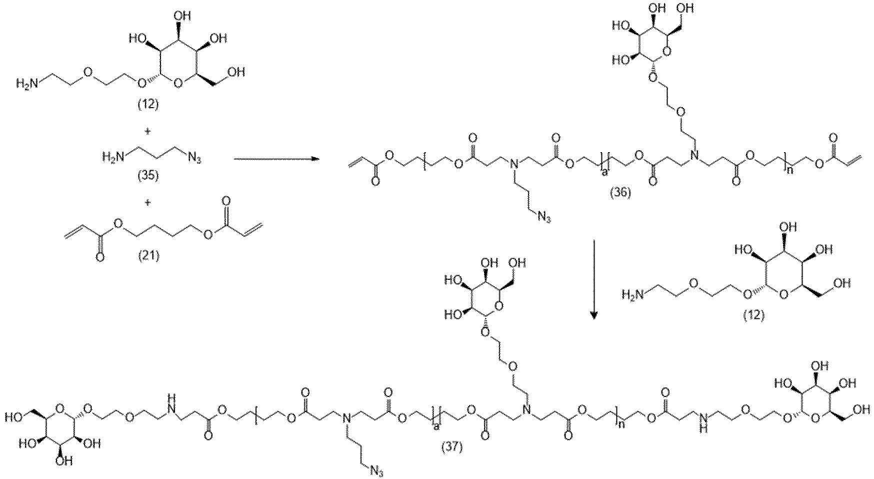

46 mg of 1,4-butanediol diacrylate (1 eq) (21), 40.7 mg of 2-(2-aminoethoxy) ethyl α-D-mannopyranoside (12) (0.85 eq) and 11.9 mg of 3-azidopropan-1-amine (35) (0.2 eq). Initially, compound 12 was dissolved with 400 µL of d-DMSO, following, the azido group and the acrylate were added. The mixture was stirred at 60 °C for 72 h. The resulting polymer (36) was added dropwise into acetone and freeze-dried. Finally, it was resuspended in DMSO-d₆. ¹H-NMR (400 MHz; DMSO-d₆): δ = 5.92 (2H, d) 4.59 (1H, 1-H), 4.09-3.98 (4H, m), 2.69 (4H, t-m), 2.55 (2H, m), 2.34 (4-6H, m), 1.59 (4H, m), 1.41 (4H, m). It was determined that the structure comprised based on 1-2 side chains of 3-azidopropan-1-amine (a), 4-5 side chains of mannosyl derivative (n) and 7-8 chains of 1,4-butanediol diacrylate.

The azido-mannosyl polymer was capped by adding 2.5 eq of 2-(2-aminoethoxy) ethyl α-D-mannopyranoside (23.8 mg) in the previously synthesized polymer. The mixture was stirred at 60 °C for 48 h. The resulting polymer (37) was dropwise into acetone and freeze-dried. Finally, it was resuspended in DMSO at 100 mg/mL, and it was characterized using NMR and UHPLC-QTOF. The chemical structure was confirmed by the absence of signals at 5.7-6.4 ppm of the terminated acrylate in the ¹H-NMR spectrum.

### 2. Click chemistry reaction

### 2.1. Click Chemistry polymer (38)

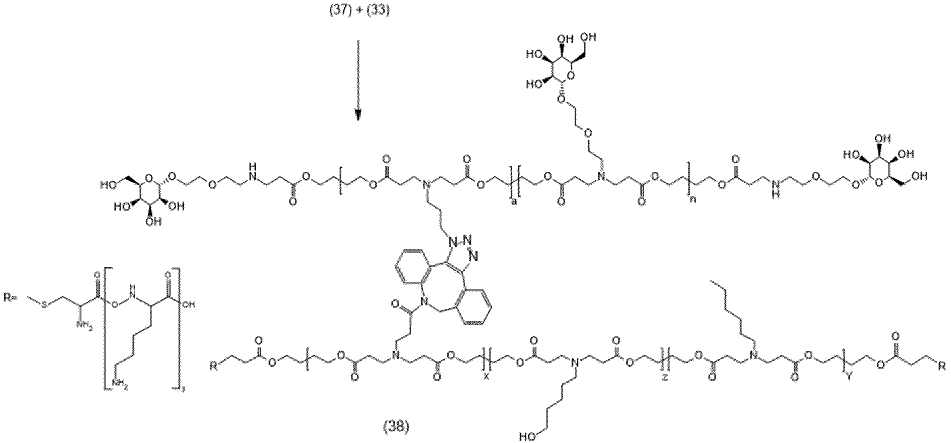

A mixture of 1.5 equivalents of C6-DBCO-CK3 (33) per 1 eq of Man2-N₃-Cap (37) was stirred at RT for 8 h in the presence of water: DMSO (1:1). Furthermore, it was dropwise into acetone, and several washes were performed. Finally, the precipitated polymer was freeze-dried and resuspended at 200 mg/ml obtaining click polymer (38). The product was analyzed by FTIR and ¹H-NMR to confirm its chemical structure. The signal at 0.82 ppm corresponding to the end chain of hexylamine was visible in the ¹H-NMR spectra, indicating the presence of C6-DBCO-CK3 in the obtained compound 38, despite its solubility in acetone, thus, meaning that the click chemistry happened. On the other hand, at 4.6 ppm the signal regarding H in C1 of α-mannose was present. Moreover, two different signals appear, meaning that we have two different types of Man2-N3-Cap, one linked via click chemistry and the other free.

### 3. Formulation of nanoparticles

For the preparation of nanoparticles, the different polymer mixtures shown in the table below were used:

| **Formulation name** | **C6-CK3** | **C6-CH3** | **C6-DBCO-CK3 (33)** | **Man2-N₃**-**Cap (37)** | **Click polymer (38)** |
|---|---|---|---|---|---|
| KH | 60% | 40% | - | - | - |
| D1 | - | 40% | 60% | - | - |
| C1 | - | 40% | - | - | 60% |

The pBAE based only on 1,4-butanediol diacrylate monomers (C6-CH3) as well as the corresponding oligopeptide-modified pBAE (C6-CK3) were obtained by end-modification of an acrylate-terminated polymer with thiol-terminated oligopeptides at a 1:2.5 molar ratio in dimethyl sulfoxide as previously described in Dosta et al., 2018.

Nanoparticles were prepared at a mass ratio 37.5:1 or 25:1 polymer:mRNA ratio, by mixing equal volumes of mRNA at 0.5 mg/mL with the D1 or C1 polymer in 12.5 mM AcONa buffer solution. The mRNA was added over the polymer solution and mixed by pipetting, followed by 15 min incubation at 25 °C. For the formation of discrete structures, this mixture (volume = Vi) was nanoprecipitated in the same volume (Vi) of DEPC (diethyl pyrocarbonate) water. After that, the same volume (Vi) of a HEPES 20 mM with (2-4) wt% sucrose solution was also added as a cryopreserving (depending on the formulation). Then, the nanoparticles were freeze-dried. The lyophilized NP-powder was kept at -20 °C until use when it was reconstituted with DEPC water (Vi).

The formulation of nanoparticles D1 and C1 maintained the same nitrogen/ phosphate(N/P) ratio as the KH nanoparticles. In terms of weight ratio, KH and D1 nanoparticles presented 25:1 ratio(w:w). However, click chemistry polymer (38) was stored at 200 mg/mL instead of 100 mg/mL, to preserve the N/P ratio once the polymer was mixed with the genetic material. The ratio for C1 nanoparticles changed to 37.5:1 (w:w, Polymer: RNA) as the presence of charged amines was exclusive to C6-DBCO-CK3.

The hydrodynamic diameter for D1 and C1 formulations were 230 and 200 nm respectively. The PDI was around 0.15 in the different tested formulations, indicating their monodispersity. Upon comparison with the KH control and between themselves, no statistically significant differences were observed in size or PDI. Although the presented size was slightly bigger >200 nm, they did not present significant differences upon comparing with KH NPs.

Subsequently, NPs were further analyzed in terms of ζ-potential. The new formulations obtained 27 mV and 26 mV for D1 and C1, respectively, presenting a significant statistical decrease compared to the 29 mV of the KH control. In terms of biological results, a decrease of 3-4 mV is not considered significant for the future interaction between the nanoparticles and the cell membranes. This decrease could be attributed to the changes in the pBAE backbone, where the C6-CK3 polymer was replaced with the C6-DBCO.

### 4. Click chemistry on the surface of already formed nanoparticles

To the nanoparticle formulation D1 as prepared previously Man2-N₃-Cap (37) was added. After this point, samples were incubated at different times. They were analyzed by DLS to observe differences in terms of size and PDI and Scattered intensity. Results were compared with the ones of a **KH** control nanoparticle formulation.

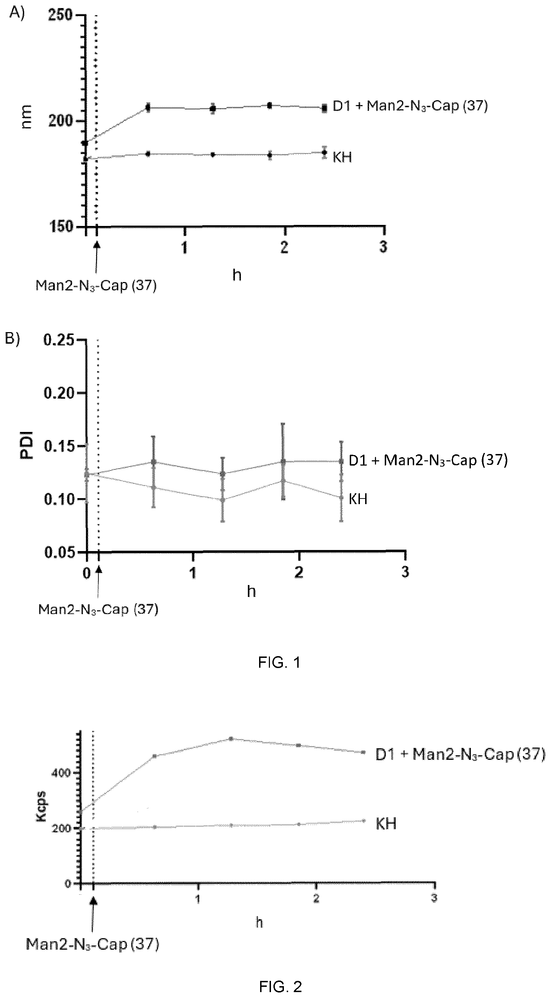

Focusing on NPs size (FIG 1A), **D1** sample nanoparticles initially measured 190 nm. Once the Man2-N₃-Cap (37) was added to the nanoparticles, an increase was observed to almost 208 nm indicating the addition of Man2-N₃-Cap (37) to the NP structure unlike the **KH** control sample, where no change in the particle size was observed. For the PDI (FIG 1B) obtained through the measures by DLS, it remained below 0.2, indicating that the particles remained monodisperse. The **KH** control did not present changes in PDI over time.

To further confirm that the click reaction was occurring at the surface of the nanoparticles, static light scattering (SLS) techniques were used. The overall scattered intensity of each sample over time was measured to assess the reaction kinetics and determine when the reaction was completed (FIG. 2).

For the **KH** control NPs, no changes were observed (remained at 200 Kcps). In contrast, once the Man2-N₃-Cap (37) was mixed into a D1 sample solution, the initial scattered intensity of 257 kcps increased up to 525 kcps (+104%), which can be attributed to an increase in the molecular mass of the nanoparticles, as a consequence of the interaction of the added building block with the already-formed NPs.

### 5. In vitro assays with click chemistry nanoparticles

### 5.1. In vitro cellular transfection and uptake

The **C1** and **D1** formulations were assayed in the transfection of JAWS II cell line.

Cells were seeded in 96 well plates at 150 000 cells mL⁻¹ 24 h before starting the experiment. Cells were incubated with 0.4 µg of mRNA coding for PureBoost^{™} EGFP mRNA/10 000 cells encapsulated within the different formulations, using KH as a positive control. Non-treated cells were used as negative controls (results correspond to 100% viability, used to normalize the absorbance of the tested samples). After some time (described in each experiment) incubation in the presence of 10% FBS, uptake and transfection efficiency were measured by flow cytometry (FC) (NovoCyte, ACEA Biosciences).

### Synthesis of polymers labeled with Cy5

To label the polymers with cyanine 5 (Cy5), the following protocol was used.16 In brief, solutions of Cy5 NHS ester in DMSO and polymers C6-CK3 and Man2-CK3 were prepared in screw cap tubes. An excess of triethylamine was added to the polymer solution and then the Cy5 solution was poured inside. The solution was stirred for 20 h at 25 1C in a dark environment and purified in a mixture of 7 : 3 diethyl ether: acetone by dropwise addition. The sample was centrifuged at 4000 rpm for 10 min to remove the solvent. This step was repeated twice. Finally, the product was dried under vacuum and dissolved at 100 mg/mL of DMSO.

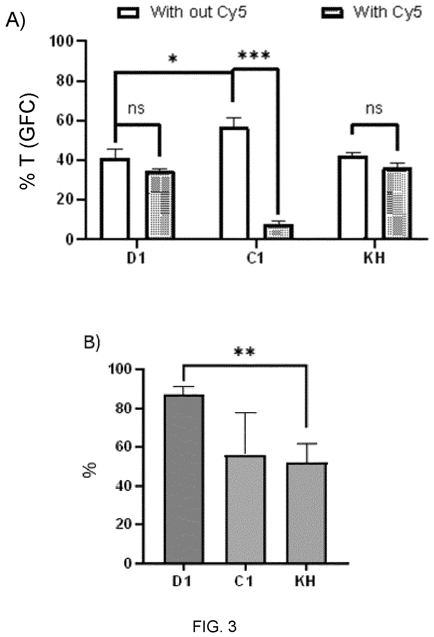

Results are shown in FIG 3. First, looking at transfection efficiency (FIG. 3A), upon comparing the flow cytometry data from the different nanoparticles, **C1** presented the highest transfection rate, ranging between 55-60%, followed by **D1** and **KH,** where both of them present 40% and 45% transfection efficiency, respectively. Differences observed between **D1** and **C1** confirm the effect of click chemistry. Regarding the uptake results (FIG. 3B), **D1** NPs demonstrate the highest uptake of NPs at 80%, followed by **C1** (the only one with mannose) and **KH,** at approximately 55% and 50%, respectively.

### 5.2. Immune activation of human monocyte-derived dendritic cells (mo-DC)

Monocytes were selected for differentiation into mo-DC, a common choice for immunization studies due to several factors. Firstly, mo-DC can be efficiently generated *in vitro* from monocytes cultured with GM-CSF and IL-4. Additionally, they exhibit functional similarities with myeloid dendritic cells, including the ability to migrate, stimulate T-cells, produce cytokines like IL-1, TNF-α, IL-12, and stimulate CD4 and CD8 T cells upon being correctly stimulated, meaning that differences in CD86 and MHC-II can be triggered due to the correct antigen presentation. Moreover, following vaccination, monocytes are recruited to the site of injection and can differentiate into mo-DCs. These mature mo-DCs take up antigens and migrate to draining lymph nodes to amplify the adaptive immune response, thus playing an active role in immune activation. Furthermore, these cells, upon their differentiation, upregulate CD206 and CD209.

Initially, monocytes were isolated from human blood and then incubated for one week with a differentiation media containing IL-4 and GM-CSF. The combination of these transcription factors triggers DC differentiation while potentially inhibiting the differentiation into macrophages or osteoclast cells. Several studies have related the degree of differentiation of mo-DC to the time of exposure of cells to IL-4 and GM-CSF.

Phenotypical changes were observed with the microscope, where the shape of monocytes was like a bean/ kidney appearance. In contrast, mo-DC are normally recognized because they present a high surface per volume ratio, moreover, their shape is irregular with different protuberances and they tend to attach to plate surfaces as can be observed.

Once monocytes were differentiated into mo-DC, nanoparticles were added to them. The formulations C1 and their controls KH and D1 were complexed with Ovalbumin (OVA) mRNA due to its effectiveness as a model immunogen for immunology research and inflammation studies. The selected testing times were 6 and 24 hours post-nanoparticle inoculation. The rationale behind selecting these times was a major issue related to pBAE nanoparticles, which, due to their structure, were capable of triggering an immune response.

The selected concentrations were chosen with the expectation of observing a dose-effect response in either CD86 or MHC-II activation. The established concentrations were 0.6, 1.5, and 3 µg of mRNA per well. The normal working concentration in the Jaws-II cell line, the cell line model used for the characterization of the NPs, was 0.6 µg of mRNA, as previously reported. Two more concentrations were tried, raising the mRNA concentration 2.5-fold and 5-fold compared with previous experiments, delving further into cell viability.

For this approach, four surface proteins coding clusters of differentiation (CD) were tracked and studied to confirm the successful differentiation and cell DC activation/maturation. The first was CD14, one of the main differentiation markers on the surface of myeloid lineage cells. Although it is not exclusively expressed in monocytes, its expression is decreased upon the differentiation to mo-DC. Next, CD11c serves as a defining marker for DCs, which also includes mo-DC. It can be also found in other cells such as monocytes, macrophages, NK cells, and in T and B cells. Their basic function is enrolled in DC antigen uptake and activation of T cells.

Following, CD86 rises as a key molecule. It is a co-stimulatory receptor expressed on the surface of DC, which is necessary for T-cell activation and stimulation. Higher levels of this molecule can lead to increased activation of DCs, facilitating T-cell activation and triggering an immune response. Specifically on mo-DC, their activation can lead to a higher expression of different cytokines, like IL-17A, which increase the inflammatory response and sensibilize mo-DC to easily detect antigens. Finally, MHC-II is another crucial molecule. Its primary function is to activate T cells, initiating the immune response. Delving into its role, MHC-II in mo-DC is an essential key for antigen-presenting function, allowing T-cell stimulation and cytokine production. Upon activation, MHC-II is upregulated, presenting antigenic fragments of extracellular proteins to T-cells and initiating the consequent specific immune cascade.

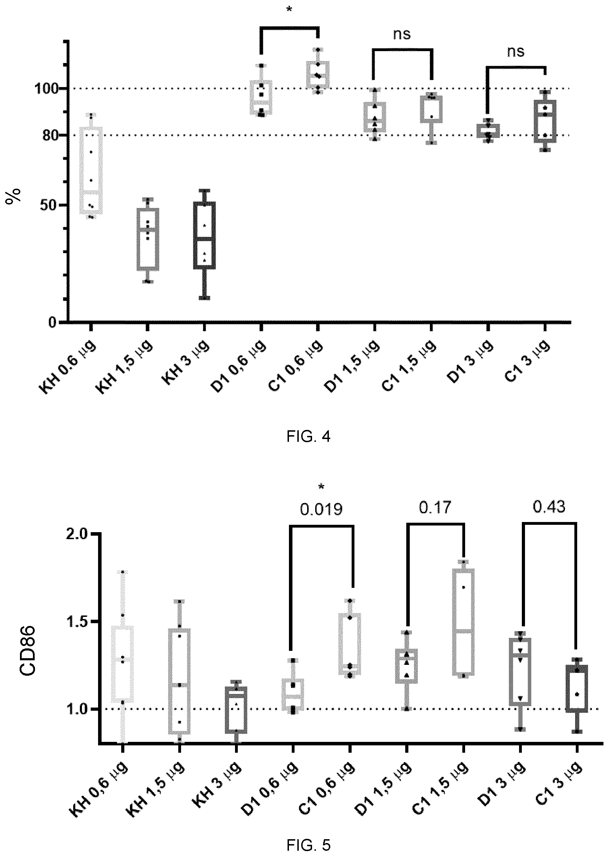

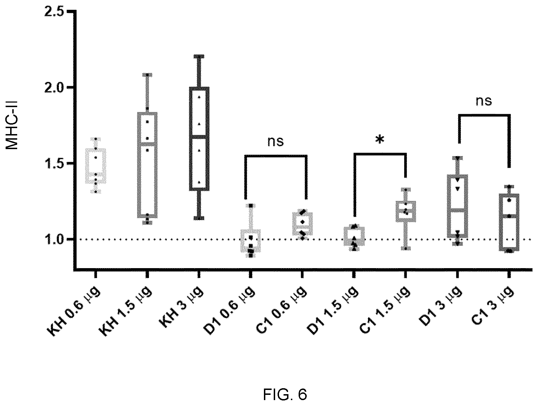

The idea about activation of mo-DC by the presence of pBAE C6 NPs, was tested at the beginning of the set-up of the experiments. The experiment consisted of the incubation during 6 hours of the 15% NPs with mo-DC. After the incubation period, cells were analyzed and compared with the negative control using FC. However, no increase in the up-regulation of CD86 and MHC-II was observed (FIG. 5 and 6). Moreover, at higher concentrations, cell viability decreased to 40% (FIG. 4). Consequently, nanoparticle removal was performed at 4 hours post-inoculation to mitigate cytotoxicity, allowing up to 50% of the nanoparticles to enter the cells, based on previously generated data for JAWS II.

From the viability point of view (FIG. 4), C1 nanoparticles present a cell viability above 85%, much more more biocompatible than KH (55-30%).

Comparing the obtained results with those previously obtained in Jaws-II, a clear trend is evident: increasing the concentration of α-mannose in the NPs improves cell viability compared to the naked nanoparticles. Surprisingly, there is no apparent reason for the enhanced cell viability observed when comparing KH and D1, which could be attributed to the presence of DBCO and the NP conformation. It can be inferred that this feature is enhanced by substituting the C6-CK3 polymer with C6-DBCO-CK3, where the hydrophobicity of DBCO building block alters the NP conformation, thereby reducing cytotoxicity.

For the maturation and activation of mo-DC, CD86 upregulation was measured (FIG. 5). Regarding C1 and D1 formulations, the functionalization with mannose in C1 NPs drives an increase in CD86 activation at low and intermediate concentrations (1.25 and 1.45 for C1; 1.1 and 1.25 for D1), with significant differences observed only at low concentrations. A substantial difference was noted between the non-mannose nanoparticles and the mannose-containing nanoparticles at intermediate concentration, achieving a p-value of 0.06. As it is clear, the addition of mannose changes the behavior of NPs, giving a dose-concentration relationship, which can be guided by the specific interaction between mannose and CD206 or CD209, allowing specific uptake. Therefore, expressing OVA antigen inside and leading to the upregulation of CD86 as is observed. At high concentrations, a decrease in cell activation was observed for C1, which could be attributed to an excess of the carbohydrate in the sample. No significant differences were observed between both formulations (D1 and C1) at high concentrations.

For the maturation and antigen presentation of mo-DC, MHC-II upregulation was measured (FIG. 6). The new polymers did not show high levels of activation at low concentrations (1 and 1.05 for D1 and C1, respectively). At middle concentration, C1 nanoparticles achieve an upregulation of 1.25, while D1 remains at 1, showing statistical differences. Finally, at higher concentrations, both formulations present upregulation of MHC-II, with both centered around 1.25. Although this disparity in the expression of MHC-II and CD86 is observed in the second generation of NPs, it is important to emphasize that MHC-II expression requires more maturation than CD86.

### Citation List

- WO2014136100

## Claims

1. A polymer, or a pharmaceutically acceptable salt thereof, which comprises a first polymer building block and a second polymer building block wherein:
a) the first and second polymer building blocks are covalently bonded through at least a common lateral chain, and
b) the first polymer building block is an oligopeptide-modified poly(beta-aminoester).

2. The polymer according to claim 1, wherein the common side chain is a biradical of formula (V) 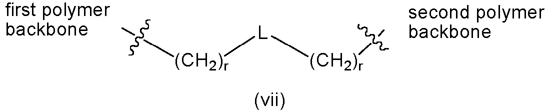 wherein:
L is -C(=O)-Z-, -NH-C(=O)-Z-, -O-C(=O)-Z-, -C(=O)-O-Z-, -C(=O)-NH-Z, -(OCH₂CH₂)ₛC(=O)-Z-, -(OCH₂CH₂)ₛNH-C(=O)-Z-, -(OCH₂CH₂)ₛO-C(=O)-Z-, -(OCH₂CH₂)ₛC(=O)-O-Z-, and -(OCH₂CH₂)ₛC(=O)-NH-Z-;
Z is a heterocycloalkenylene comprising at least two nitrogen atoms and two fused rings;
and each r is independently an integer from 1 to 10. each r is independently an integer from 1 to 10.

3. The polymer according to any one of claims 1-2, wherein the first polymer building block is an oligo-modified poly(beta-aminoester) of formula (I): 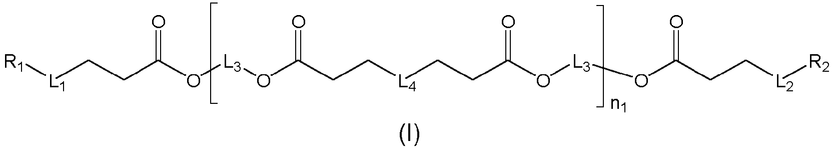 wherein:
each L₃ is independently selected from the group consisting of alkylene, alkenylene, heteroalkylene, heteroalkenylene, arylene, and heteroarylene; or alternatively
at least one occurrence of L₃ is a biradical of formula (i₁): 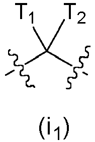 wherein:
T₁ is a radical of formula (ii₁): 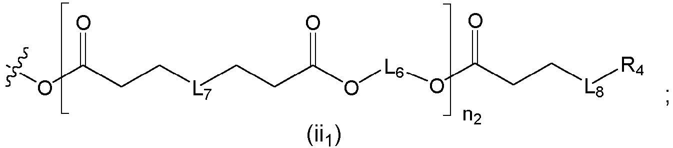
T₂ is selected from the group consisting of H, alkyl, and a radical of formula (ii₁);
and the remaining L₃ groups are independently selected at each occurrence from the group consisting of alkylene, alkenylene, heteroalkylene, heteroalkenylene, arylene, and heteroarylene;
each L₄ and L₇ are independently selected from the group consisting of a biradical of formula (iii₁) and formula (iv₁): 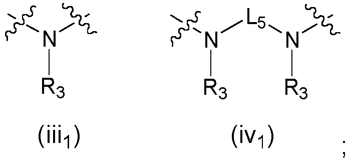
each L₁, L₂, and L₈ are independently selected from the group consisting of O, S, NRₓ, a bond, and a biradical of formula (v₁); 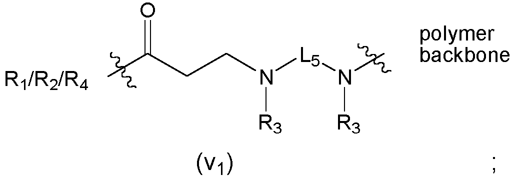
each L₅ and L₆ are independently selected from the group consisting of alkylene, alkenylene, heteroalkylene, heteroalkenylene, arylene, and heteroarylene;
R₁, R₂ and each R₄ are independently selected from the group consisting of an oligopeptide and R_{y}; wherein at least one of R₁, R₂ and R₄ is an oligopeptide;
each R₃ is independently selected from the group consisting of a bond, hydrogen, halogen, alkyl, cycloalkyl, alkenyl, cycloalkenyl, heteroalkyl, heterocycloalkyl, acyl, aryl, heteroaryl, a polyalkylene glycol, -(CH₂)ₚOH, -(CH₂)₂(OCH₂CH₂)_{q}OH, -(CH₂)ₚNH₂, and -(CH₂)₂(OCH₂CH₂)_{q}NH₂;
each Rₓ and R_{y} is independently selected from the group consisting of hydrogen, halogen, alkyl, cycloalkyl, alkenyl, cycloalkenyl, heteroalkyl, heterocycloalkyl, acyl, aryl, and heteroaryl; and
n₁ and n₂ are independently an integer from 5 to 1000; p is an integer from 1 to 20; and q, is an integer from 1 to 10,
provided that at least one R₃ in any of L₁, L₂, L₃, L₄, L₇ and L₈ is a bond which binds the first building block to one of the ends of the at least one common lateral chain.

4. The polymer according to claim 3, wherein each L₃ is independently selected from the group consisting of alkylene, alkenylene, heteroalkylene, heteroalkenylene, arylene, and heteroarylene.

5. The polymer according to any one of claims 3-4, wherein L₁ and L₂ are a bond, and R₁ and R₂ are independently an oligopeptide.

6. The polymer according to any one of claims 1-5, wherein the second polymer building block is functionalized with one or more active ingredients, cell-targeting ligands, diagnostic agents, imaging agents, or combinations thereof.

7. The polymer according to any one of claims 1-6, wherein the second polymer building block is selected from the group consisting of a poly(beta-aminoester), a polyalkylene glycol, a polyester, a polyacrylate, a halogen-containing polymer, a poly(4-N-Bocvinylaniline) (PBVA) or polystyrene (PS).

8. The polymer according to claim 7, wherein the second polymer building block is a poly(beta-aminoester) of formula (II): 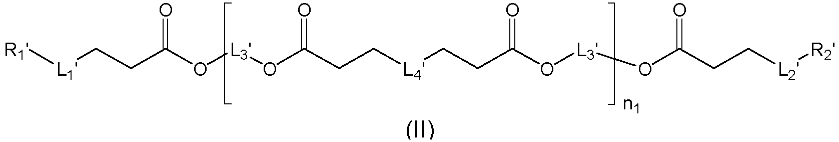 wherein:
each L₃' is independently selected from the group consisting of alkylene, alkenylene, heteroalkylene, heteroalkenylene, arylene, and heteroarylene; or alternatively
at least one occurrence of L₃' is a biradical of formula (i₁'): 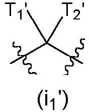 wherein:
T₁' is a radical of formula (ii₁'): 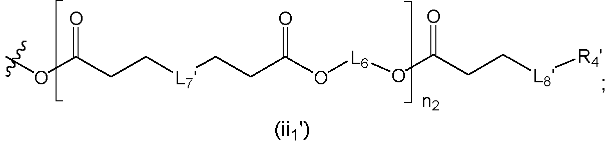
T₂' is selected from the group consisting of H, alkyl and a radical of formula (ii₁');
and the remaining L₃' groups are independently selected at each occurrence from the group consisting of alkylene, alkenylene, heteroalkylene, heteroalkenylene, arylene, and heteroarylene;
each L₄' and L₇' are independently selected from the group consisting of a biradical of formula (iii₁') and of formula (iv₁'): 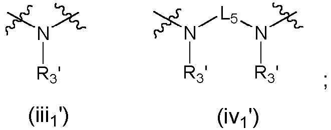
each L₁', L₂', and L₈' are independently selected from the group consisting of O, S, NRₓ, a bond, and a biradical of formula (v₁'); 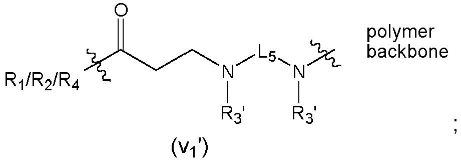
each L₅ and L₆ are independently selected from the group consisting of alkylene, alkenylene, heteroalkylene, heteroalkenylene, arylene, and heteroarylene;
R₁', R₂' and each R₄' are independently selected from the group consisting of an oligopeptide, R_{y}, and a moiety selected from the group consisting of an active ingredient moiety, a cell-targeting moiety, a diagnostic agent moiety, and an imaging agent moiety;
each R₃' is independently selected from the group consisting of a bond, hydrogen, halogen, alkyl, cycloalkyl, alkenyl, cycloalkenyl, heteroalkyl, heterocycloalkyl, acyl, aryl, heteroaryl, a polyalkylene glycol, -(CH₂)ₚOH, -(CH₂)₂(OCH₂CH₂)_{q}OH, -(CH₂)ₚNH₂, -(CH₂)₂(OCH₂CH₂)_{q}NH₂, and a moiety selected from the group consisting of an active ingredient moiety, a cell-targeting moiety, a diagnostic agent moiety, and an imaging agent moiety;
each Rₓ and R_{y} is independently selected from the group consisting of hydrogen, halogen, alkyl, cycloalkyl, alkenyl, cycloalkenyl, heteroalkyl, heterocycloalkyl, acyl, aryl, and heteroaryl; and
n₁ and n₂ are independently an integer from 5 to 1000; p is an integer from 1 to 20; and q, is an integer from 1 to 10;
provided that the second polymer building block comprises at least an active ingredient, a cell-targeting ligand, a diagnostic agent, or an imaging agent; and that at least one R₃' in any of L₁', L₂', L₃', L₄', L₇' and L₈' is a bond which binds the second building block to one of the ends of the at least one common lateral chain.

9. The polymer according to claim 8, wherein each L₃' is independently selected from the group consisting of alkylene, alkenylene, heteroalkylene, heteroalkenylene, arylene, and heteroarylene.

10. A nanoparticle comprising the polymer as defined in any one of claims 1-9.

11. The nanoparticle according to claim 10, which further comprises an active ingredient, a diagnostic agent, an imaging agent or a combination thereof.

12. A pharmaceutical composition comprising the polymer as defined in any one of claims 1-9 or the nanoparticle as defined in any one of claims 10-11, and a therapeutic effective amount of an active ingredient, a cell-targeting ligand, a diagnostic agent, an imaging agent or a combination thereof, together with one or more pharmaceutically acceptable excipients.

13. A cosmetic composition comprising the polymer as defined in any one of claims 1-9 or the nanoparticle as defined in any one of claims 10-11, and a cosmetically effective amount of an active ingredient, together with one or more cosmetically acceptable excipients.

14. A polymer as defined in any one of claims 1-9 or the nanoparticle as defined in any one of claims 10-11, for use as a delivery system.

15. A nanoparticle as defined in any one of the claims 10-11 or the pharmaceutical composition as defined in claim 12, for use in therapy, diagnosis or cell-targeting.
